# EUROPEAN PATENT SPECIFICATION

(11) **EP 0 799 205 B1**
(45) Date of publication and mention of the grant of the patent: **08.09.1999**
(21) Application number: 95944511.5
(22) Date of filing: 21.12.1995
(51) Int. Cl.: C07D 213/30, A61K 31/44

(54) **1,4,4-(TRISUBSTITUTED)CYCLOHEX-1-ENE DERIVATIVES AS PDE IV- AND TNF-INHIBITORS**
1,4,4-(TRISUBSTITUIERTE)CYCLOHEX-1-EN-DERIVATE ALS PDE IV- UND TNF-HEMMER
MONOMERES DE 1,4,4-(TRISUBSTITUE)CYCLOHEX-1-ENE UTILISES COMME INHIBITEURS DE PDE IV ET TNF

(30) Priority: 23.12.1994 US 363168
(43) Date of publication of application: 08.10.1997
(73) Proprietor: SMITHKLINE BEECHAM CORPORATION, King of Prussia, PA 19406-0939 (US)
(72) Inventor: CHRISTENSEN, Siegfried, B., IV, Philadelphia, PA 19103 (US); KARPINSKI, Joseph, M., Pottstown, PA 19464 (US); RYAN, M., Dominic, Pottstown, PA 19464 (US); BENDER, Paul, E., Cherry Hill, NJ 08003 (US)
(74) Representative: Rutter, Keith, Dr.
(86) International application number: US9516694
(87) International publication number: WO9620175

(56) References cited:
- WO-A-93/19720
- WO-A-93/19748
- WO-A-94/14800

## Description

### Field of Invention

The present invention relates to novel 1,4,4-(trisubstituted)cyclohex-1-ene monomers and related compounds, pharmaceutical compositions containing these compounds, and their use in treating allergic and inflammatory diseases and for inhibiting the production of Tumor Necrosis Factor (TNF).

### Background of the Invention

Bronchial asthma is a complex, multifactorial disease characterized by reversible narrowing of the airway and hyperreactivity of the respiratory tract to external stimuli.

Identification of novel therapeutic agents for asthma is made difficult by the fact that multiple mediators are responsible for the development of the disease. Thus, it seems unlikely that eliminating the effects of a single mediator will have a substantial effect on all three components of chronic asthma. An alternative to the "mediator approach" is to regulate the activity of the cells responsible for the pathophysiology of the disease.

One such way is by elevating levels of cAMP (adenosine cyclic 3',5'-monophosphate). Cyclic AMP has been shown to be a second messenger mediating the biologic responses to a wide range of hormones, neurotransmitters and drugs; [Krebs Endocrinology Proceedings of the 4th International Congress Excerpta Medica, 17-29, 1973]. When the appropriate agonist binds to specific cell surface receptors, adenylate cyclase is activated, which converts Mg⁺²-ATP to cAMP at an accelerated rate.

Cyclic AMP modulates the activity of most, if not all, of the cells that contribute to the pathophysiology of extrinsic (allergic) asthma. As such, an elevation of cAMP would produce beneficial effects including: 1) airway smooth muscle relaxation, 2) inhibition of mast cell mediator release, 3) suppression of neutrophil degranulation, 4) inhibition of basophil degranulation, and 5) inhibition of monocyte and macrophage activation. Hence, compounds that activate adenylate cyclase or inhibit phosphodiesterase should be effective in suppressing the inappropriate activation of airway smooth muscle and a wide variety of inflammatory cells. The principal cellular mechanism for the inactivation of cAMP is hydrolysis of the 3'-phosphodiester bond by one or more of a family of isozymes referred to as cyclic nucleotide phosphodiesterases (PDEs).

It has now been shown that a distinct cyclic nucleotide phosphodiesterase (PDE) isozyme, PDE IV, is responsible for cAMP breakdown in airway smooth muscle and inflammatory cells. [Torphy, "Phosphodiesterase Isozymes: Potential Targets for Novel Anti-asthmatic Agents" in New Drugs for Asthma, Barnes, ed. IBC Technical Services Ltd., 1989]. Research indicates that inhibition of this enzyme not only produces airway smooth muscle relaxation, but also suppresses degranulation of mast cells, basophils and neutrophils along with inhibiting the activation of monocytes and neutrophils. Moreover, the beneficial effects of PDE IV inhibitors are markedly potentiated when adenylate cyclase activity of target cells is elevated by appropriate hormones or autocoids, as would be the case *in vivo.* Thus PDE IV inhibitors would be effective in the asthmatic lung, where levels of prostaglandin E₂ and prostacyclin (activators of adenylate cyclase) are elevated. Such compounds would offer a unique approach toward the pharmacotherapy of bronchial asthma and possess significant therapeutic advantages over agents currently on the market.

The compounds of this invention also inhibit the production of Tumor Necrosis Factor (TNF), a serum glycoprotein. Excessive or unregulated TNF production has been implicated in mediating or exacerbating a number of diseases including rheumatoid arthritis, rheumatoid spondylitis, osteoarthritis, gouty arthritis and other arthritic conditions; sepsis, septic shock, endotoxic shock, gram negative sepsis, toxic shock syndrome, adult respiratory distress syndrome, cerebral malaria, chronic pulmonary inflammatory disease, silicosis, pulmonary sarcoidosis, bone resorption diseases, reperfusion injury, graft vs. host reaction, allograft rejections, fever and myalgias due to infection, such as influenza, cachexia secondary to infection or malignancy, cachexia secondary to human acquired immune deficiency syndrome (AIDS), AIDS, ARC (AIDS related complex), keloid formation, scar tissue formation, Crohn's disease, ulcerative colitis, or pyresis, in addition to a number of autoimmune diseases, such as multiple sclerosis, autoimmune diabetes and systemic lupus erythematosis.

AIDS results from the infection of T lymphocytes with Human Immunodeficiency Virus (HIV). At least three types or strains of HIV have been identified, i.e., HIV-1, HIV-2 and HIV-3. As a consequence of HIV infection, T-cell-mediated immunity is impaired and infected individuals manifest severe opportunistic infections and/or unusual neoplasms. HIV entry into the T lymphocyte requires T lymphocyte activation. Viruses such as HIV-1 or HIV-2 infect T lymphocytes after T cell activation and such virus protein expression and/or replication is mediated or maintained by such T cell activation. Once an activated T lymphocyte is infected with HIV, the T lymphocyte must continue to be maintained in an activated state to permit HIV gene expression and/or HIV replication.

Cytokines, specifically TNF, are implicated in activated T-cell-mediated HIV protein expression and/or virus replication by playing a role in maintaining T lymphocyte activation. Therefore, interference with cytokine activity such as by inhibition of cytokine production, notably TNF, in an HIV-infected individual aids in limiting the maintenance of T cell activation, thereby reducing the progression of HIV infectivity to previously uninfected cells which results in a slowing or elimination of the progression of immune dysfunction caused by HIV infection. Monocytes, macrophages, and related cells, such as kupffer and glial cells, have also been implicated in maintenance of the HIV infection. These cells, like T cells, are targets for viral replication and the level of viral replication is dependent upon the activation state of the cells. [See Rosenberg *et al.,* The Immunopathogenesis of HIV Infection, Advances in Immunology, Vol. 57, 1989]. Monokines, such as TNF, have been shown to activate HIV replication in monocytes and/or macrophages [See Poli *et al.,* Proc. Natl. Acad. Sci., 87:782-784, 1990], therefore, inhibition of monokine production or activity aids in limiting HIV progression as stated above for T cells.

TNF has also been implicated in various roles with other viral infections, such as the cytomegalovirus (CMV), influenza virus, adenovirus, and the herpes virus for similar reasons as those noted.

TNF is also associated with yeast and fungal infections. Specifically *Candida albicans* has been shown to induce TNF production *in vitro* in human monocytes and natural killer cells. [See Riipi *et al,,* Infection and Immunity, 58(9):2750-54, 1990; and Jafari *et al.,* Journal of Infectious Diseases, 164:389-95, 1991. See also Wasan *et al.,* Antimicrobial Agents and Chemotherapy, 35,(10):2046-48, 1991; and Luke *et al.,* Journal of Infectious Diseases, 162:211-214,1990].

The ability to control the adverse effects of TNF is furthered by the use of the compounds which inhibit TNF in mammals who are in need of such use. There remains a need for compounds which are useful in treating TNF-mediated disease states which are exacerbated or caused by the excessive and/or unregulated production of TNF.

### Summary of the Invention

The first aspect of this invention relates to compounds of Formula (I) : 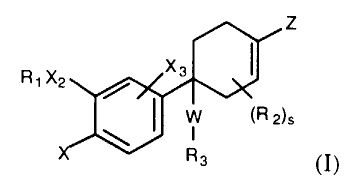 wherein:
R₁ is -(CR₄R₅)ₙC(O)O(CR₄R₅)ₘR₆, -(CR₄R₅)ₙC(O)NR₄(CR₄R₅)ₘR₆,-(CR₄R₅)ₙO(CR₄R₅)ₘR₆, or -(CR₄R₅)ᵣR₆ wherein the alkyl moieties are unsubstituted or substituted with one or more halogens;
m is 0 to 2;
n is 0 to 4;
r is 0 to 6;
R₄ and R₅ are independently hydrogen or a C₁₋₂ alkyl;
R₆ is hydrogen, methyl, hydroxyl, aryl, halo substituted aryl, aryloxyC₁₋₃ alkyl, halo substituted aryloxyC₁₋₃ alkyl, indanyl, indenyl, C₇₋₁₁ polycycloalkyl, tetrahydrofuranyl, furanyl, tetrahydropyranyl, pyranyl, tetrahydrothienyl, thienyl, tetrahydrothiopyranyl, thiopyranyl, C₃₋₆ cycloalkyl, or a C₄₋₆ cycloalkyl containing one or two unsaturated bonds, wherein the cycloalkyl or heterocyclic moiety may be unsubstituted or substituted by 1 to 3 methyl groups, one ethyl group or an hydroxyl group;
provided that:
a) when R₆ is hydroxyl, then m is 2; or
b) when R₆ is hydroxyl, then r is 2 to 6; or
c) when R₆ is 2-tetrahydropyranyl, 2-tetrahydrothiopyranyl, 2-tetrahydrofuranyl, or 2-tetrahydrothienyl, then m is 1 or 2; or
d) when R₆ is 2-tetrahydropyranyl, 2-tetrahydrothiopyranyl, 2-tetrahydrofuranyl, or 2-tetrahydrothienyl, then r is 1 to 6;
e) when n is 1 and m is 0, then R₆ is other than H in -(CR₄R₅)ₙO(CR₄R₅)ₘR₆;
X is YR₂, fluorine, NR₄R₅, or formyl amine;
Y is O or S(O)_{m'};
m' is 0, 1, or 2;
X₂ is O or NR₈;
X₃ is hydrogen or X;
R₂ is -CH₃ or -CH₂CH₃ unsubstituted or substituted by 1 or more halogens;
s is 0 to 4;
W is alkyl of 2 to 6 carbons, alkenyl of 2 to 6 carbons or alkynyl of 2 to 6 carbons;
R₃ is COOR₁₄, C(O)NR₄R₁₄ or R₁₅;
Z is S(O)_{m'}R₉, OS(O)₂R₉, OR₉, OC(O)NR₇R₇, OC(O)(O)_{q}R₇, O(CR₄R₅)ₙOR₉, or NR₉R₉;
q is 0 or 1;
R₇ is hydrogen or R₉;
R₈ is hydrogen or C₁₋₄ alkyl unsubstituted or substituted by one to three fluorines, or when R₈ and R₁₀ are as -NR₈R₁₀ they may together with the nitrogen form a a 5 to 7 membered ring comprised only of carbon atoms or carbon atoms and at least one heteroatom selected from O/N/or S;
R₉ is C₁₋₁₀ alkyl, C₂₋₁₀ alkenyl, C₃₋₇cycloalkyl, C₄₋₆ cycloalkenyl, aryl, arylalkyl, heteroaryl, heteroarylalkyl, each of which may be unsubstituted or substituted by one or more fluorine atoms, or two R₉ terms appearing as NR₉R₉ may together with the nitrogen form a 5 to 7 membered ring comprised only of carbon atoms or carbon atoms and at least one heteroatom selected from O/N/or S;
R₁₀ is OR₈ or R₈;
R₁₁ is C₁₋₄ alkyl unsubstituted or substituted by one to three fluorines;
R₁₂ is R₁₃, C₃-C₇ cycloalkyl, or an unsubstituted or substituted aryl or heteroaryl group selected from the group consisting of (2-, 3- or 4-pyridyl), pyrimidyl, pyrazolyl, (1- or 2-imidazolyl), pyrrolyl, piperazinyl, piperidinyl, morpholinyl, furanyl, (2- or 3-thienyl), quinolinyl, naphthyl, and phenyl;
R₁₃ is a substituted or unsubstituted heteroaryl group selected from the group consisting of oxazolidinyl, oxazolyl, thiazolyl, pyrazolyl, triazolyl, tetrazolyl, imidazolyl, imidazolidinyl, thiazolidinyl, isoxazolyl, oxadiazolyl, and thiadiazolyl, and where R₁₃ is substituted on R₁₂ or R₁₃ the rings are connected through a carbon atom and each second R₁₃ ring may be unsubstituted or substituted by one or two C₁₋ ₂ alkyl groups unsubstituted or substituted on the methyl with 1 to 3 fluoro atoms;
R₁₄ is hydrogen or R₁₅; or when R₁₀ and R₁₄ are as NR₁₀R₁₄ they may together with the nitrogen form a 5 to 7 membered ring comprised only of carbon atoms or carbon atoms and at least one heteroatom selected from O/N/or S;
R₁₅ is -(CR₄R₅)ₜR₁₂ or C₁₋₆ alkyl wherein the R₁₂ or C₁₋₆ alkyl group is unsubstituted or substituted by one or more times by methyl or ethyl unsubstituted or substituted by one to three fluorines, -F, -Br, -Cl, -NO₂, -Si(R₄)₂, -NR₈R₁₀,-C(O)R₈, -C(O)OR₈, -O(CH₂)_{q}R₈, -CN, -C(O)NR₈R₁₀, -O(CH₂)_{q}C(O)NR₈R₁₀,-O(CH₂)_{q}C(O)R₈, -NR₁₀C(O)NR₈R₁₀, -NR₁₀C(O)R₈, -NR₁₀C(O)OR₉, -NR₁₀C(O)R₁₃, -C(NR₁₀)NR₈R₁₀, -C(NCN)NR₈R₁₀, -C(NCN)SR₁₁, -NR₁₀C(NCN)SR₁₁, -NR₁₀C(NCN)NR₁₀R₈, -NR₁₀S(O)₂R₉, -S(O)_{m'}R₁₁, -NR₁₀C(O)C(O)NR₈R₁₀, -NR₁₀C(O)C(O)R₁₀, or R₁₃;
t is 0, 1, or 2;
provided that:
f) when q is 1 in OC(O)(O)_{q}R₇ , then R₇ is not hydrogen;
g) R₇ is not C₁₋₄ alkyl unsubstituted or substituted by one to three fluorines; or the pharmaceutically acceptable salts thereof.

Another set of compounds of this invention are represented by Formula (II): 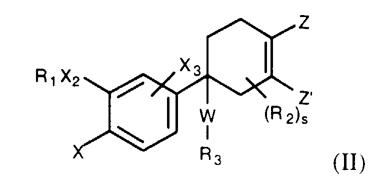 wherein:
R₁ is -(CR₄R₅)ₙC(O)O(CR₄R₅)ₘR₆, -(CR₄R₅)ₙC(O)NR₄(CR₄R₅)ₘR₆,-(CR₄R₅)ₙO(CR₄R₅)ₘR₆, or -(CR₄R₅)ᵣR₆ wherein the alkyl moieties are unsubstituted or substituted with one or more halogens;
m is 0 to 2;
n is 0 to 4;
r is 0 to 6;
R₄ and R₅ are independently hydrogen or a C₁₋₂ alkyl;
R₆ is hydrogen, methyl, hydroxyl, aryl, halo substituted aryl, aryloxyC₁₋₃ alkyl, halo substituted aryloxyC₁₋₃ alkyl, indanyl, indenyl, C₇₋₁₁ polycycloalkyl, tetrahydrofuranyl, furanyl, tetrahydropyranyl, pyranyl, tetrahydrothienyl, thienyl, tetrahydrothiopyranyl, thiopyranyl, C₃₋₆ cycloalkyl, or a C₄₋₆ cycloalkyl containing one or two unsaturated bonds, wherein the cycloalkyl or heterocyclic moiety may be unsubstituted or substituted by 1 to 3 methyl groups, one ethyl group or an hydroxyl group;
provided that:
a) when R₆ is hydroxyl, then m is 2; or
b) when R₆ is hydroxyl, then r is 2 to 6; or
c) when R₆ is 2-tetrahydropyranyl, 2-tetrahydrothiopyranyl, 2-tetrahydrofuranyl, or 2-tetrahydrothienyl, then m is 1 or 2; or
d) when R₆ is 2-tetrahydropyranyl, 2-tetrahydrothiopyranyl, 2-tetrahydrofuranyl, or 2-tetrahydrothienyl, then r is 1 to 6;
e) when n is 1 and m is 0, then R₆ is other than H in -(CR₄R₅)ₙO(CR₄R₅)ₘR₆;
X is YR₂, fluorine, NR₄R₅, or formyl amine;
Y is O or S(O)_{m'};
m' is 0, 1, or 2;
X₂ is O or NR₈;
X₃ is hydrogen or X;
R₂ is -CH₃ or -CH₂CH₃ unsubstituted or substituted by 1 or more halogens;
s is 0 to 4;
W is alkyl of 2 to 6 carbons, alkenyl of 2 to 6 carbons or alkynyl of 2 to 6 carbons;
R₃ is COOR₁₄, C(O)NR₄R₁₄ or R₁₅;
Z is NHR₁₄, S(O)_{m'}R₉, OS(O)₂R₉, OR₉, OC(O)NR₇R₇, OC(O)(O)_{q}R₇, O(CR₄R₅)ₙOR₉, or NR₉R₉;
Z' is C(Y')R₁₄, C(O)OR₁₄, C(Y')NR₁₀R₁₄, C(NR₁₀)NR₁₀R₁₄, CN, C(NOR₈)R₁₄, C(NOR₁₄)R₈, C(NR₈)NR₁₀R₁₄, C(NR₁₄)NR₈R₈ C(NCN)NR₁₀R₁₄, C(NCN)SR₁₁, (2-, 4- or 5-imidazolyl), (3-, 4- or 5-pyrazolyl), (4-or 5-triazolyl[1,2,3]), (3- or 5-triazolyl[1,2,4]), (5-tetrazolyl), (2-, 4- or 5-oxazolyl), (3-, 4- or 5-isoxazolyl), (3- or 5-oxadiazolyl[1,2,4]), (2-oxadiazolyl[1,3,4]), (2-thiadiazolyl[1,3,4]), (2-, 4-, or 5-thiazolyl), (2-, 4-, or 5-oxazolidinyl), (2-, 4-, or 5-thiazolidinyl), or (2-, 4-, or 5-imidazolidinyl); wherein all of the heterocylic ring systems may be optionally substituted one or more times by R₁₄;
Y' is O or S;
q is 0 or 1;
R₇ is hydrogen or R₉;
R₈ is hydrogen or C₁₋₄ alkyl unsubstituted or substituted by one to three fluorines, or when R₈ and R₁₀ are as -NR₈R₁₀ they may together with the nitrogen form a a 5 to 7 membered ring comprised only of carbon atoms or carbon atoms and at least one heteroatom selected from O, N, or S;
R₉ is C₁₋₁₀ alkyl, C₂₋₁₀ alkenyl, C₃₋₇cycloalkyl, C₄₋₆ cycloalkenyl, aryl, arylalkyl, heteroaryl, heteroarylalkyl, each of which may be unsubstituted or substituted by one or more fluorine atoms, or two R₉ terms appearing as NR₉R₉ may together with the nitrogen form a 5 to 7 membered ring comprised only of carbon atoms or carbon atoms and at least one heteroatom selected from O/N/or S;
R₁₀ is OR₈ or R₈;
R₁₁ is C₁₋₄ alkyl unsubstituted or substituted by one to three fluorines;
R₁₂ is R₁₃, C₃-C₇ cycloalkyl, or an unsubstituted or substituted aryl or heteroaryl group selected from the group consisting of (2-, 3- or 4-pyridyl), pyrimidyl, pyrazolyl, (1- or 2-imidazolyl), pyrrolyl, piperazinyl, piperidinyl, morpholinyl, furanyl, (2- or 3-thienyl), quinolinyl, naphthyl, and phenyl;
R₁₃ is a substituted or unsubstituted heteroaryl group selected from the group consisting of oxazolidinyl, oxazolyl, thiazolyl, pyrazolyl, triazolyl, tetrazolyl, imidazolyl, imidazolidinyl, thiazolidinyl, isoxazolyl, oxadiazolyl, and thiadiazolyl, and where R₁₃ is substituted on R₁₂ or R₁₃ the rings are connected through a carbon atom and each second R₁₃ ring may be unsubstituted or substituted by one or two C₁₋ ₂ alkyl groups unsubstituted or substituted on the methyl with 1 to 3 fluoro atoms;
R₁₄ is hydrogen or R₁₅; or when R₁₀ and R₁₄ are as NR₁₀R₁₄ they may together with the nitrogen form a 5 to 7 membered ring comprised only of carbon atoms or carbon atoms and at least one heteroatom selected from O, N, or S;
R₁₅ is -(CR₄R₅)ₜR₁₂ or C₁₋₆ alkyl wherein the R₁₂ or C₁₋₆ alkyl group is unsubstituted or substituted by one or more times by methyl or ethyl unsubstituted or substituted by one to three fluorines, -F, -Br, -Cl, -NO₂, -Si(R₄)₂, -NR₈R₁₀,-C(O)R₈, -C(O)OR₈, -O(CH₂)_{q}R₈, -CN, -C(O)NR₈R₁₀, -O(CH₂)_{q}C(O)NR₈R₁₀,-O(CH₂)_{q}C(O)R₈, -NR₁₀C(O)NR₈R₁₀, -NR₁₀C(O)R₈, -NR₁₀C(O)OR₉, -NR₁₀C(O)R₁₃, -C(NR₁₀)NR₈R₁₀, -C(NCN)NR₈R₁₀, -C(NCN)SR₁₁, -NR₁₀C(NCN)SR₁₁, -NR₁₀C(NCN)NR₁₀R₈, -NR₁₀S(O)₂R₉, -S(O)ₘ'R₁₁, -NR₁₀C(O)C(O)NR₈R₁₀, -NR₁₀C(O)C(O)R₁₀, or R₁₃;
t is 0, 1, or 2;
provided that:
f) when q is 1 in OC(O)(O)_{q}R₇ , then R₇ is not hydrogen;
g) R₇ is not C₁₋₄ alkyl unsubstituted or substituted by one to three fluorines;
or the pharmaceutically acceptable salts thereof.

This invention also relates to the pharmaceutical compositions comprising a compound of the invention and a pharmaceutically acceptable carrier or diluent.

The invention also relates to a compound of the invention for use in the mediation or inhibition of the enzymatic activity (or catalytic activity) of PDE IV in mammals, including humans. The administration to a mammal in need thereof is illustrated below.

The invention further provides to a compound of the invention for use in the treatment of allergic and inflammatory disease in a mammal, including humans.

The invention also provides to a compound of the invention for use in a method for the treatment of asthma in a mammal, including humans, in need thereof, an effective amount of a compound of the invention.

This invention also relates to to a compound of the invention for use in the inhibition of TNF production in a mammal, including humans. There is also provided a compound of the invention for use in the prophylactic treatment or prevention of certain TNF mediated disease states amenable thereto.

This invention also relates to a compound of the invention for use in a method for treating a human afflicted with a human immunodeficiency virus (HIV).

Compounds of the invention are also useful in the treatment of additional viral infections, where such viruses are sensitive to upregulation by TNF or will elicit TNF production *in vivo.*

In addition, compounds of the invention are useful in treating yeast and fungal infections, where such yeast and fungi are sensitive to upregulation by TNF or will elicit TNF production *in vivo.*

### Detailed Description of the Invention

This invention also relates to a compound of the invention for use in the mediation or inhibition of the enzymatic activity (or catalytic activity) of PDE IV in a mammal and for the inhibition of the production of TNF in a mammal.

Phosphodiesterase IV inhibitors are useful in the treatment of a variety of allergic and inflammatory diseases including: asthma, chronic bronchitis, atopic dermatitis, urticaria, allergic rhinitis, allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock and adult respiratory distress syndrome. In addition, PDE IV inhibitors are useful in the treatment of diabetes insipidus and central nervous system disorders such as depression and multi-infarct dementia.

The viruses contemplated for treatment herein are those that produce TNF as a result of infection, or those which are sensitive to inhibition, such as by decreased replication, directly or indirectly, by the TNF inhibitors of the invention. Such viruses include, but are not limited to HIV-1, HIV-2 and HIV-3, cytomegalovirus (CMV), influenza, adenovirus and the Herpes group of viruses, such as, but not limited to, *Herpes zoster* and *Herpes simplex.*

This invention more specifically relates to a compound of the invention for use in the treatment of human immunodeficiency virus (HIV).

The compounds of this invention may also be used in association with the veterinary treatment of animals, other than in humans, in need of inhibition of TNF production. TNF mediated diseases for treatment, therapeutically or prophylactically, in animals include disease states such as those noted above, but in particular viral infections. Examples of such viruses include, but are not limited to feline immunodeficiency virus (FIV) or other retroviral infection such as equine infectious anemia virus, caprine arthritis virus, visna virus, maedi virus and other lentiviruses.

The compounds of this invention are also useful in treating yeast and fungal infections, where such yeast and fungi are sensitive to upregulation by TNF or will elicit TNF production *in vivo.* A preferred disease state for treatment is fungal meningitis. Additionally, the compounds of the invention may be administered in conjunction with other drugs of choice for systemic yeast and fungal infections. Drugs of choice for fungal infections, include but are not limited to the class of compounds called the polymixins, such as Polymycin B, the class of compounds called the imidazoles, such as clotrimazole, econazole, miconazole, and ketoconazole; the class of compounds called the triazoles, such as fluconazole, and itranazole, and the class of compound called the Amphotericins, in particular Amphotericin B and liposomal Amphotericin B.

The compounds of the invention may also be used for inhibiting and/or reducing the toxicity of an anti-fungal, anti-bacterial or anti-viral agent by administering an effective amount of a compound of the invention to a mammal in need of such treatment. Preferably, a compound of the invention is administered for inhibiting or reducing the toxicity of the Amphotericin class of compounds, in particular Amphotericin B.

The term "C₁₋₃ alkyl", "C₁₋₄ alkyl", "C₁₋₆ alkyl" or "alkyl" groups as used herein is meant to include both straight or branched chain radicals of 1 to 10, unless the chain length is limited thereto, including, but not limited to methyl, ethyl, n-propyl, isopropyl, n-butyl, see-butyl, isobutyl, *tert*-butyl, and the like.

"Alkenyl" means both straight or branched chain radicals of 1 to 6 carbon lengths, unless the chain length is limited thereto, including but not limited to vinyl, 1-propenyl, 2-propenyl, 2-propynyl, or 3-methyl-2-propenyl.

The term "cycloalkyl" or "cycloalkyl alkyl" means groups of 3-7 carbon atoms, such as cyclopropyl, cyclopropylmethyl, cyclopentyl, or cyclohexyl.

"Aryl" or "aralkyl", unless specified otherwise, means an aromatic ring or ring system of 6-10 carbon atoms, such as phenyl, benzyl, phenethyl, or naphthyl. Preferably the aryl is monocyclic, i.e, phenyl. The alkyl chain is meant to include both straight or branched chain radicals of I to 4 carbon atoms.

"Heteroaryl" means an aromatic ring system containing one or more heteroatoms, such as imidazolyl, triazolyl, oxazolyl, pyridyl, pyrimidyl, pyrazolyl, pyrrolyl, furanyl, or thienyl.

"Halo" means all halogens, i.e., chloro, fluoro, bromo, or iodo.

"Inhibiting the production of IL-1" or "inhibiting the production of TNF" means:
a) a decrease of excessive *in vivo* IL-1 or TNF levels, respectively, in a human to normal levels or below normal levels by inhibition of the *in vivo* release of IL-1 by all cells, including but not limited to monocytes or macrophages;
b) a down regulation, at the translational or transcriptional level, of excessive *in vivo* IL- I or TNF levels, respectively, in a human to normal levels or below normal levels; or
c) a down regulation, by inhibition of the direct synthesis of IL-1 or TNF levels as a postranslational event.

The phrase "TNF mediated disease or disease states" means any and all disease states in which TNF plays a role, either by production of TNF itself, or by TNF causing another cytokine to be released, such as but not limited to IL-1 or IL-6. A disease state in which IL-1, for instance is a major component, and whose production or action, is exacerbated or secreted in response to TNF, would therefore be considered a disease state mediated by TNF. As TNF-β (also known as lymphotoxin) has close structural homology with TNF-α (also known as cachectin), and since each induces similar biologic responses and binds to the same cellular receptor, both TNF-α and TNF-β are inhibited by the compounds of the present invention and thus are herein referred to collectively as "TNF" unless specifically delineated otherwise. Preferably TNF-α is inhibited.

"Cytokine" means any secreted polypeptide that affects the functions of cells, and is a molecule which modulates interactions between cells in immune, inflammatory, or hematopoietic responses. A cytokine includes, but is not limited to, monokines and lymphokines regardless of which cells produce them.

The cytokine inhibited by the present invention for use in the treatment of a HIV-infected human must be a cytokine which is implicated in (a) the initiation and/or maintenance of T cell activation and/or activated T cell-mediated HIV gene expression and/or replication, and/or (b) any cytokine-mediated disease associated problem such as cachexia or muscle degeneration. Preferrably, his cytokine is TNF-α.

All of the compounds of the invention are useful in the method of inhibiting the production of TNF, preferably by macrophages, monocytes or macrophages and monocytes, in a mammal, including humans, in need thereof. All of the compounds of the invention are useful in the method of inhibiting or mediating the enzymatic or catalytic activity of PDE IV and in treatment of disease states mediated thereby.

Pharmaceutically acceptable salts of the instant compounds, where they can be prepared, are also intended to be covered by this invention. These salts will be ones which are acceptable in their application to a pharmaceutical use. By that it is meant that the salt will retain the biological activity of the parent compound and the salt will not have untoward or deleterious effects in its application and use in treating diseases.

Preferred compounds are as follows:

When R₁ is an alkyl substituted by 1 or more halogens, the halogens are preferably fluorine and chlorine, more preferably a C₁₋₄ alkyl substituted by 1 or more fluorines. The preferred halo-substituted alkyl chain length is one or two carbons, and most preferred are the moieties -CF₃, -CH₂F, -CHF₂, -CF₂CHF₂,-CH₂CF₃, and -CH₂CHF₂. Preferred R₁ substitutents for the compounds of the invention are CH₂-cyclopropyl, CH₂-C₅₋₆ cycloalkyl, C₄₋₆ cycloalkyl unsubstituted or substituted with OH, C₇₋₁₁ polycycloalkyl, (3- or 4-cyclopentenyl), phenyl, tetrahydrofuran-3-yl, benzyl or C₁₋₂ alkyl unsubstituted or substituted by 1 or more fluorines, -(CH₂)₁₋₃C(O)O(CH₂)₀₋₂CH₃, -(CH₂)₁₋₃O(CH₂)₀₋₂CH₃, and -(CH₂)₂₋₄OH.

When R₁ term contains the moiety (CR₄R₅), the R₄ and R₅ terms are independently hydrogen or alkyl. This allows for branching of the individual methylene units as (CR₄R₅)ₙ or (CR₄R₅)ₘ; each repeating methylene unit is independent of the other, e.g., (CR₄R₅)ₙ wherein n is 2 can be -CH₂CH(-CH₃)-, for instance. The individual hydrogen atoms of the repeating methylene unit or the branching hydrocarbon can unsubstituted or be substituted by fluorine independent of each other to yield, for instance, the preferred R₁ substitutions, as noted above.

When R₁ is a C₇₋₁₁ polycycloalkyl, examples are bicyclo[2.2.1]-heptyl, bicyclo[2.2.2]octyl, bicyclo[3.2.]octyl, tricyclo[5.2.1.0^{2,6}]decyl, etc. additional examples of which are described in Saccamano *et al.,* WO 87/06576, published 5 November 1987.

W is preferably alkyl, alkenyl or alkynyl of 2 to 4 carbon atoms, and where it is alkenyl or alkynyl, that one or two double or triple bonds be present. It is most preferred that W is ethynyl or 1,3-butadiynyl.

Z is preferably OR₉, O(S)₂R₉ and NR₉R₉. Most preferred are OR₉, O(S)₂R₉.

Preferred Z' is COOR₁₄.

Preferred X groups for the invention are those wherein X is YR₂ and Y is oxygen. The preferred X₂ group for the invention is that wherein X₂ is oxygen. The preferred X₃ group for The is that wherein X₃ is hydrogen. Preferred R₂ groups, where applicable, is a C₁₋₂ alkyl unsubstituted or substituted by 1 or more halogens. The halogen atoms are preferably fluorine and chlorine, more preferably fluorine. More preferred R₂ groups are those wherein R₂ is methyl, or the fluoro-substituted alkyls, specifically a C₁₋₂ alkyl, such as a -CF₃, -CHF₂, or -CH₂CHF₂ moiety. Most preferred are the -CHF₂ and -CH₃ moieties.

Preferred R₁₅ moieties include unsubstituted or substituted -(CH₂)₀₋₂(2-, 3-or 4-pyridyl), (CH₂)₁₋₂(2-imidazolyl), (CH₂)₂(4-morpholinyl), (CH₂)₂(4-piperazinyl), (CH₂)₁₋₂(2-thienyl), (CH₂)₁₋₂(4-thiazolyl), unsubstituted or substituted pyrimidinyl, and substituted or unsubstituted (CH₂)_{O-2}phenyl.

Preferred rings when R₈ and R₁₁ in the moiety -NR₈R₁₁ and when R₉ in the mointy NR₉R₉ together with the nitrogen to which they are attached form a 5 to 7 membered ring comprised of carbon or carbon and at least one heteroatom selected from O, N, or S include, but are not limited to 1-imidazolyl, 2-(R₈)-1-imidazolyl, 1-pyrazolyl, 3-(R₈)-1-pyrazolyl, 1-triazolyl, 2-triazolyl, 5-(R₈)-1-triazolyl, 5-(R₈)-2-triazolyl, 5-(R₈)-1-tetrazolyl, 5-(R₈)-2-tetrazolyl, 1-tetrazolyl, 2-tetrazloyl, morpholinyl, piperazinyl, 4-(R₈)-1-piperazinyl, or pyrrolyl ring.

Preferred rings when R₈ and R₁₄ in the moiety -NR₈R₁₄ together with the nitrogen to which they are attached may form a 5 to 7 membered ring comprised of carbon or carbon and at least one heteroatom selected from O, N, or S include, but are not limited to 1-imidazolyl, 1-pyrazolyl, 1-triazolyl, 2-triazolyl, 1-tetrazolyl, 2-tetrazolyl, morpholinyl, piperazinyl, and pyrrolyl. The respective rings may be additionally substituted, where applicable, on an available nitrogen or carbon by the moiety R7 as described herein for Formula (I). Illustrations of such carbon substitutions includes, but is not limited to, 2-(R₁₅)-1-imidazolyl, 4-(R₁₅)-1-imidazolyl, 5-(R₁₅)-1-imidazolyl, 3-(R₁₅)-1-pyrazolyl, 4-(R₁₅)-1-pyrazolyl, 5-(R₁₅)-1-pyrazolyl, 4-(R₁₅)-2-triazolyl, 5-(R₁₅)-2-triazolyl, 4-(R₁₅)-1-triazolyl, 5-(R₁₅)-1-triazolyl, 5-(R₁₅)-1-tetrazolyl, and 5-(R₁₅)-2-tetrazolyl. Applicable nitrogen substitution by R₇ includes, but is not limited to, 1-(R₁₅)-2-tetrazolyl, 2-(R₁₅)-1-tetrazolyl, 4-(R₁₅)-1-piperazinyl. Where applicable, the ring may be substituted one or more times by R₁₅.

Preferred groups for NR₈R₁₄ which contain a heterocyclic ring are 5-(R₁₄)-1-tetrazolyl, 2-(R₁₄)-1-imidazolyl, 5-(R₁₄)-2-tetrazolyl, 4-(R₁₄)-1-piperazinyl, or 4-(R₁₅)-1-piperazinyl.

Preferred rings for R₁₃ include (2-, 4- or 5-imidazolyl), (3-, 4- or 5-pyrazolyl), (4- or 5-triazolyl[1,2,3]), (3- or 5-triazolyl[1,2,4]), (5-tetrazolyl), (2-, 4-or 5-oxazolyl), (3-, 4- or 5-isoxazolyl), (3- or 5-oxadiazolyl[1,2,4]), (2-oxadiazolyl[1,3,4]), (2-thiadiazolyl[1,3,4]), (2-, 4-, or 5-thiazolyl), (2-, 4-, or 5-oxazolidinyl), (2-, 4-, or 5-thiazolidinyl), or (2-, 4-, or 5-imidazolidinyl).

When the R₁₅ group is unsubstituted or substituted by a heterocyclic ring such as imidazolyl, pyrazolyl, triazolyl, tetrazolyl, or thiazolyl, the heterocyclic ring itself may be unsubstituted or substituted by R₈ either on an available nitrogen or carbon atom, such as 1-(R₈)-2-imidazolyl, 1-(R₈)-4-imidazolyl, 1-(R₈)-5-imidazolyl, 1-(R₈)-3-pyrazolyl, 1-(R₈)-4-pyrazolyl, 1-(R₈)-5-pyrazolyl, 1-(R₈)-4-triazolyl, or 1-(R₈)-5-triazolyl. Where applicable, the ring may be substituted one or more times by R₈.

Preferred are those compounds of Formula (I) wherein R₁ is -CH₂-cyclopropyl, -CH₂-C₅₋₆ cycloalkyl, -C₄₋₆ cycloalkyl unsubstituted or substituted by OH, tetrahydrofuran-3-yl, (3- or 4-cyclopentenyl), benzyl or -C₁₋₂ alkyl unsubstituted or substituted by 1 or more fluorines, and -(CH₂)₂₋₄ OH; R₂ is methyl or fluoro-substituted alkyl, W is ethynyl or 1,3-butadiynyl; R₁₅ is an unsubstituted or substituted aryl or heteroaryl ring, X is YR₂, Z is OR₉ or O(S)₂R₉ and Z' is COOR₁₄.

Most preferred are those compounds wherein R₁ is -CH₂-cyclopropyl, cyclopentyl, 3-hydroxycyclopentyl, methyl or CF₂H; X is YR₂; Y is oxygen; X₂ is oxygen; X₃ is hydrogen; and R₂ is CF₂H or methyl, W is ethynyl or 1,3-butadiynyl, and R₃ is a substituted or unsubstituted pyrimidinyl ring.

The exemplified compounds are:
4-(3-cyclopentyloxy-4-methoxyphenyl)-4-(4-pyridylethynyl)cyclohex-1-en-1-yl trifluoromethylsulfonate, and
4-(3-cyclopentyloxy-4-methoxyphenyl)-4-(4-pyridylethynyl)-1-methoxycyclohex-1-ene.

It will be recognized that some of the compounds of the invention may exist in both racemic and optically active forms; some may also exist in distinct diastereomeric forms possessing distinct physical and biological properties. All of these compounds are considered to be within the scope of the present invention.

Pharmaceutically acceptable salts are prepared in a standard manner. The parent compound, dissolved in a suitable solvent, is treated with an excess of an organic or inorganic acid, in the case of acid addition salts of a base, or an excess of organic or inorganic base where the molecule contains a COOH for example.

Pharmaceutical compositions of the present invention comprise a pharmaceutical carrier or diluent and some amount of a compound of the invention. The compound may be present in an amount to effect a physiological response, or it may be present in a lesser amount such that the user will need to take two or more units of the composition to effect the treatment intended. These compositions may be made up as a solid, liquid or in a gaseous form. Or one of these three forms may be transformed to another at the time of being administered such as when a solid is delivered by aerosol means, or when a liquid is delivered as a spray or aerosol.

The nature of the composition and the pharmaceutical carrier or diluent will, of course, depend upon the intended route of administration, for example parenterally, topically, orally or by inhalation.

For topical administration the pharmaceutical composition will be in the form of a cream, ointment, liniment, lotion, pastes, aerosols, and drops suitable for administration to the skin, eye, ear, or nose.

For parenteral administration the pharmaceutical composition will be in the form of a sterile injectable liquid such as an ampule or an aqueous or non-aqueous liquid suspension.

For oral administration the pharmaceutical composition will be in the form of a tablet, capsule, powder, pellet, atroche, lozenge, syrup, liquid, or emulsion.

When the pharmaceutical composition is employed in the form of a solution or suspension, examples of appropriate pharmaceutical carriers or diluents include: for aqueous systems, water; for non-aqueous systems, ethanol, glycerin, propylene glycol, corn oil, cottonseed oil, peanut oil, sesame oil, liquid parafins and mixtures thereof with water; for solid systems, lactose, kaolin and mannitol; and for aerosol systems, dichlorodifluoromethane, chlorotrifluoroethane and compressed carbon dioxide. Also, in addition to the pharmaceutical carrier or diluent, the instant compositions may include other ingredients such as stabilizers, antioxidants, preservatives, lubricants, suspending agents, viscosity modifiers and the like, provided that the additional ingredients do not have a detrimental effect on therapeutic action of the instant compositions.

The pharmaceutical preparations thus described are made following the conventional techniques of the pharmaceutical chemist as appropriate to the desired end product.

In these compositions, the amount of carrier or diluent will vary but preferably will be the major proportion of a suspension or solution of the active ingredient. When the diluent is a solid it may be present in lesser, equal or greater amounts than the solid active ingredient.

Usually a compound of formula I is administered to a subject in a composition comprising a nontoxic amount sufficient to produce an inhibition of the symptoms of a disease in which leukotrienes are a factor. Topical formulations will contain between about 0.01 to 5.0% by weight of the active ingredient and will be applied as required as a preventative or curative agent to the affected area. When employed as an oral, or other ingested or injected regimen, the dosage of the composition is selected from the range of from 50 mg to 1000 mg of active ingredient for each administration. For convenience, equal doses will be administered 1 to 5 times daily with the daily dosage regimen being selected from about 50 mg to about 5000 mg.

No unacceptable toxicological effects are expected when these compounds are administered in accordance with the present invention.

The following examples are given to further illustrate the described invention. These examples are intented solely for illustrating the invention and should not be read to limit the invention in any manner. Reference is made to the claims for what is reserved to the inventors hereunder.

### Methods Of Preparation

### Synthetic Scheme(s) With Textual Description

Compounds of the invention may be prepared by the processes disclosed herein which comprise reacting a terminal acetylene, wherein Z represents Z as defined in relation to the invention or a group convertible to Z, as, *e.g.,* compound 1-Scheme 1, with an appropriate halide, R₃X, wherein R₃ represents R₃ as defined in relation to the invention or a group convertible to R₃, in the presence of a suitable catalyst, such as a copper (I) halide and a bivalent or zerovalent palladium compound in the presence of, *e.g.,* triphenylphosphine, in a suitable solvent, such as an amine, as in the procedure of Brandsma *et al.* (Syn. Comm., 1990, 20, 1889), provides a compound of the Formula 2-Scheme 1. Compounds of the Formula 1-Scheme 1 may be prepared by procedures analogous to those described in copending U.S. patent applications 07/862114, 07/968806, and PCT application PCT/US93/02230 which are now published as International Application, publication number WO 93/19720.

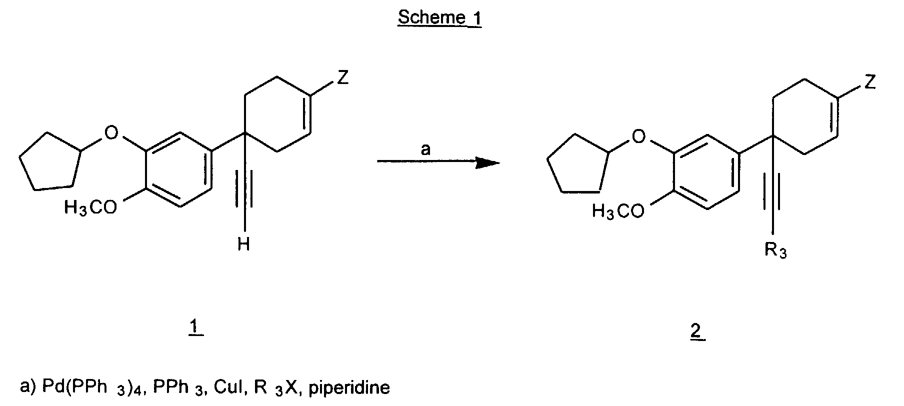

Alternatively, compounds of the invention, wherein Z and R₃ represent Z and R₃ as defined in relation to the invention or a group convertible to Z or R₃, may be prepared from the corresponding ketones as, *e.g.,* compound 1-Scheme 2, by the synthetic procedures described in in copending U.S. patent applications 07/862114, 07/968806, and PCT application PCT/US93/02230 which are now published as International Application, publication number WO 93/19720; syntheses of such ketone starting materials are described in prior filed co-pending U.S. applications 07/862,083, 07/968,753 and PCT/US93/01990 designating the United States and filed 05 March 1993 (WIPO publication No. WO 93/19748).

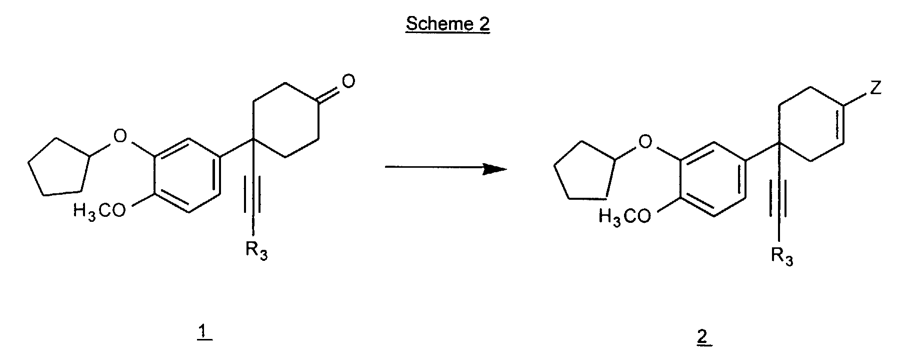

Alternatively, oxidative carbonylation of a terminal acetylene as, *e.g.,* compound 1-Scheme 3, using an appropriate metal salt, such as a copper salt with a catalytic amount of a palladium salt, in the presence of a suitable base as an acid trap, such as sodium acetate, in a suitable alcohol, such as methanol, as in the method of Tsuji *et al.* (Tet. Lett., 1980, 21, 849), then provides the compound of the Formula 2-Scheme 3; such compounds may then be converted to other compounds of the invention by manipulation of the ketone as described above and by independent manipulation of the carboxylic ester moiety using standard transesterification or amidation conditions. Syntheses of such ketone starting materials are described in prior filed co-pending U.S. applications 07/862,083, 07/968,753 and PCT/US93/01990 designating the United States and filed 05 March 1993 (WIPO publication No. WO 93/19748).

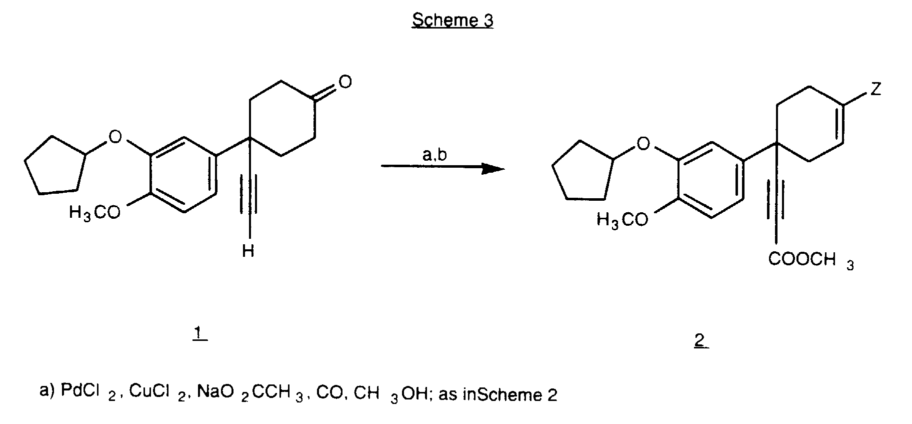

Likewise, oxidative carbonylation of a terminal acetylene as, *e.g.*, compound 1-Scheme 4, wherein Z represents Z as defined in relation to the invention or a group convertible to Z, using an appropriate metal salt, such as a copper salt with a catalytic amount of a palladium salt, in the presence of a suitable base as an acid trap, such as sodium acetate, in a suitable alcohol, such as methanol, as in the method of Tsuji *et al.* (Tet. Lett., 1980, 21, 849), then provides the compound of the Formula 2-Scheme 4; such compounds may then be converted to other compounds of the invention by manipulation of the carboxylic ester moiety using standard transesterification or amidation conditions.

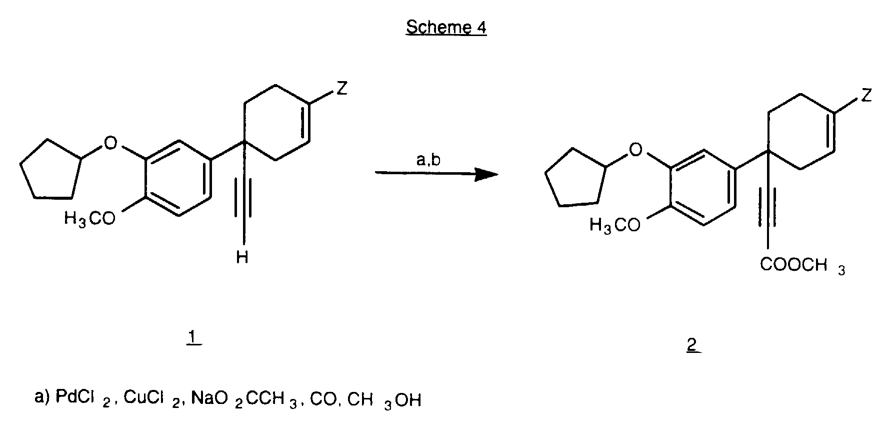

Similarly, compounds of Formula (II) may be prepared by the processes disclosed herein which comprise reacting a terminal acetylene, wherein Z represents Z as defined in relation to the invention or a group convertible to Z, as, *e.g.,* compound 1-Scheme 1, with an appropriate halide, R₃X, wherein R₃ represents R₃ as defined in relation to the invention or a group convertible to R₃, in the presence of a suitable catalyst, such as a copper (I) halide and a bivalent or zerovalent palladium compound in the presence of, *e.g.,* triphenylphosphine, in a suitable solvent, such as an amine, as in the procedure of Brandsma *et al.* (Syn. Comm., 1990, 20, 1889), provides a compound of the Formula 2-Scheme 5. Compounds of the Formula 1-Scheme 5 may be prepared by procedures analogous to those described in copending U.S. patent applications 07/862114, 07/968806, and PCT application PCT/US93/02230 which are now published as International Application publication number WO 93/19720.

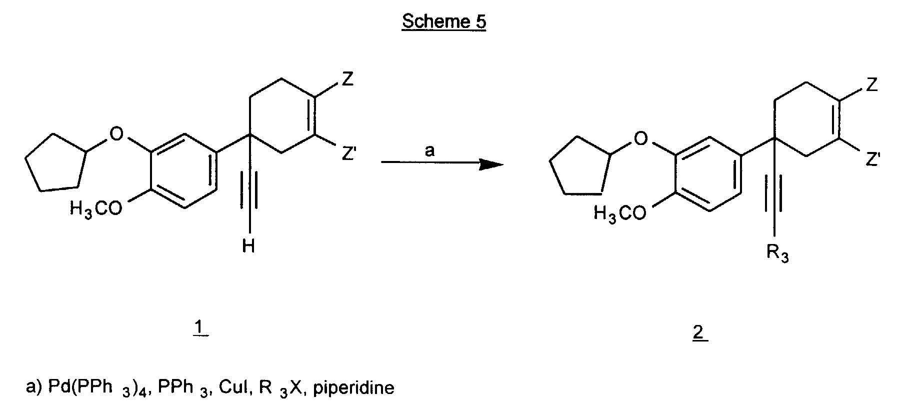

Alternatively, compounds of the Formula (II), wherein Z and R₃ represent Z and R₃ as defined in relation to Formula (II) or a group convertible to Z or R₃, may be prepared from the corresponding ketones as, *e.g.,* compound 1-Scheme 6, by the synthetic procedures described in copending U.S. patent applications 07/862114, 07/968806, and PCT application PCT/US93/02230 which are now published as International Application publication number WO 93/19720; a process for making the ketones is disclosed in prior filed co-pending U.S. applications 07/862,083, 07/968,753 and PCT/US93/01990 designating the United States and filed 05 March 1993 (WIPO publication No. WO 93/19748).

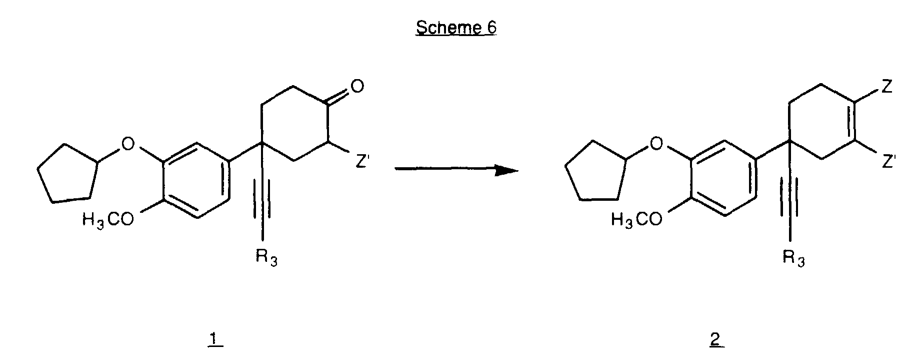

Alternatively, oxidative carbonylation of a terminal acetylene as, *e.g.,* compound 1-Scheme 7, using an appropriate metal salt, such as a copper salt with a catalytic amount of a palladium salt, in the presence of a suitable base as an acid trap, such as sodium acetate, in a suitable alcohol, such as methanol, as in the method of Tsuji *et al.* (Tet. Lett., 1980, 21, 849), then provides the compound of the Formula 2-Scheme 7; such compounds may then be converted to other compounds of the Formula (II) by manipulation of the ketone as described above and by independent manipulation of the carboxylic ester moiety using standard transesterification or amidation conditions. Syntheses of such ketone starting materials are described in prior filed co-pending U.S. applications 07/862,083, 07/968,753 and PCT/US93/01990 designating the United States and filed 05 March 1993 (WIPO publication No. WO 93/19748).

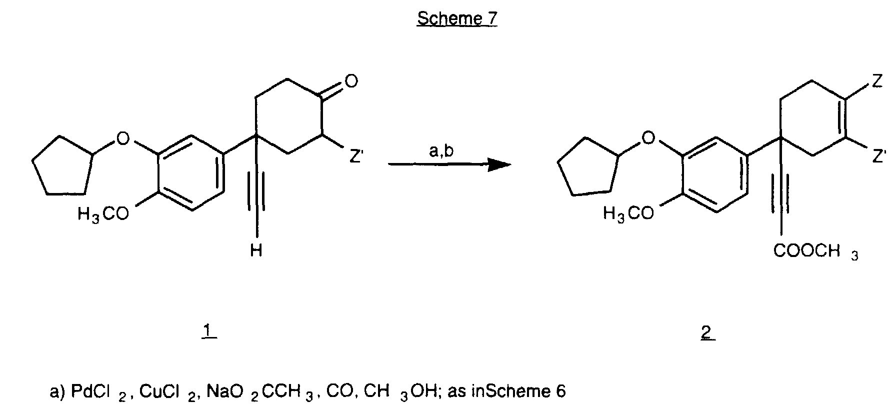

Likewise, oxidative carbonylation of a terminal acetylene as, *e.g.,* compound 1-Scheme 8, wherein Z and Z' represent Zand Z' as defined in relation to Formula (II) or a group convertible to Z or Z', using an appropriate metal salt, such as a copper salt with a catalytic amount of a palladium salt, in the presence of a suitable base as an acid trap, such as sodium acetate, in a suitable alcohol, such as methanol, as in the method of Tsuji *et al.* (Tet. Lett., 1980, 21, 849), then provides the compound of the Formula 2-Scheme 48; such compounds may then be converted to other compounds of the Formula (II) by manipulation of the carboxylic ester moiety using standard transesterification or amidation conditions.

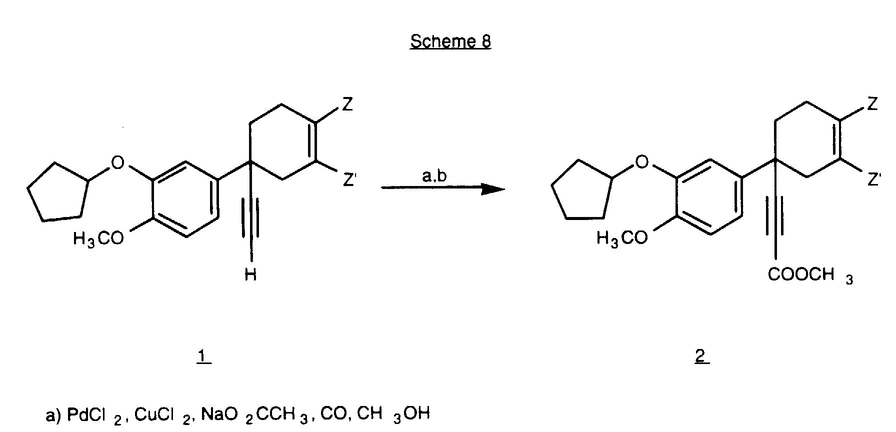

Preparation of the remaining compounds of the invention and (II) may be accomplished by procedures analogous to those described above and in the Examples, *infra.*

It will be recognized that some compounds of the invention and (II) may exist in distinct diastereomeric forms possessing distinct physical and biological properties; such isomers may be separated by standard chromatographic methods.

### Synthetic Examples

### Example 1

### Preparation of 4-(3-cyclopentyloxy-4-methoxyphenyl)-4-(4-pyridylethynyl)cyclohex1-en-1-yl trifluoromethylsulfonate

To a solution of diisopropylamine (1.95 mL, 13.9 mmol) in tetrahydrofuran (12 mL) at 0°C under an argon atmosphere is added n-butyllithium (5.8 mL of 2.5M solution, 14.15 mmol), the resulting solution is stirred for 25 min and then is cooled to -78°C. To this is added a solution of 4-(3-cyclopentyloxy-4-methoxyphenyl)-4-(4-pyridylethynyl)cyclohexan-1-one (2.59 g, 6.64 mmol, prepared by the procedures described in a co-pending U.S. patent application filed on even date herewith and identified as P50283) in tetrahydrofuran (9 mL). The resulting mixture is stirred at - 78°C for 2 h, at which time N-phenyl-trifluoromethylsulfonimide (4.98 g, 13.9 mmol) is added. The mixture is allowed to warm slowly to room temperature and after 5 h, the mixture is poured into water and extracted with methylene chloride. The organic extract is dried (potassium carbonate) and concentrated under reduced pressure. The residue is purified by flash chromatography.

### Example 2

### Preparation of 4-(3-cyclopentyloxy-4-methoxyphenyl)-4-(4-pyridylethynyl)-1-methoxycyclohex-1-ene

To a solution of 4-(3-cyclopentyloxy-4-methoxyphenyl)-4-(4-pyridylethynyl)-cyclohexan-1-one (0.37 g, 0.96 mmol) in dimethylformamide (3 mL) at 0°C under an argon atmosphere is added potassium t-butoxide (0.11 g, 0.96 mmol) and, 0.5 h later, dimethyl sulfate (0.09 mL, 0.96 mmol). After 5 min, ammonium chloride is added, the mixture is extracted three times with ether, the organic extract is washed three times with water, once with brine, is dried (magnesium sulfate) and is evaporated. Purification by flash chromatography provides the title compound.

### UTILITY EXAMPLES

### EXAMPLE A

### Inhibitory effect of compounds of the invention on in vitro TNF production by human monocytes

The inhibitory effect of compounds of the invention on *in vitro* TNF production by human monocytes may be determined by the protocol as described in Badger *et al.,* EPO published Application 0 411 754 A2, February 6, 1991, and in Hanna, WO 90/15534, December 27, 1990.

### EXAMPLE B

Two models of endotoxic shock have been utilized to determine *in vivo* TNF activity for the compounds of the invention. The protocol used in these models is described in Badger *et al.,* EPO published Application 0 411 754 A2, February 6, 1991, and in Hanna, WO 90/15534, December 27, 1990.

The compound of Example 1 herein demonstrated a positive *in vivo* response in reducing serum levels of TNF induced by the injection of endotoxin.

### EXAMPLE C

### Isolation of PDE Isozymes

The phosphodiesterase inhibitory activity and selectivity of the compounds of the invention can be determined using a battery of five distinct PDE isozymes. The tissues used as sources of the different isozymes are as follows: 1) PDE Ib, porcine aorta; 2) PDE Ic, guinea-pig heart; 3) PDE III, guinea-pig heart; 4) PDE IV, human monocyte; and 5) PDE V (also called "Ia"), canine trachealis. PDEs Ia, Ib, Ic and III are partially purified using standard chromatographic techniques [Torphy and Cieslinski, Mol. Pharmacol., 37:206-214, 1990]. PDE IV is purified to kinetic homogeneity by the sequential use of anion-exchange followed by heparin-Sepharose chromatography [Torphy *et al.,* J. Biol. Chem., 267:1798-1804, 1992].

Phosphodiesterase activity is assayed as described in the protocol of Torphy and Cieslinski, Mol. Pharmacol., 37:206-214, 1990. Positive IC₅₀'s in the nanomolar to µM range for compounds of the workings examples described herein for the invention have been demonstrated.

## Claims

1. A compound of Formula (I) 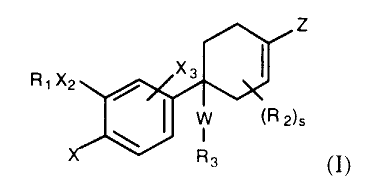 wherein:
R₁ is -(CR₄R₅)ₙC(O)O(CR₄R₅)ₘR₆, -(CR₄R₅)ₙC(O)NR₄(CR₄R₅)ₘR₆,-(CR₄R₅)ₙO(CR₄R₅)ₘR₆, or -(CR₄R₅)ᵣR₆ wherein the alkyl moieties are unsubstituted or substituted with one or more halogens;
m is 0 to 2;
n is 0 to 4;
r is 0 to 6;
R₄ and R₅ are independently hydrogen or a C₁₋₂ alkyl;
R₆ is hydrogen, methyl, hydroxyl, aryl, halo substituted aryl, aryloxyC₁₋₃ alkyl, halo substituted aryloxyC₁₋₃ alkyl, indanyl, indenyl, C₇₋₁₁ polycycloalkyl, tetrahydrofuranyl, furanyl, tetrahydropyranyl, pyranyl, tetrahydrothienyl, thienyl, tetrahydrothiopyranyl, thiopyranyl, C₃₋₆ cycloalkyl, or a C₄₋₆ cycloalkyl containing one or two unsaturated bonds, wherein the cycloalkyl or heterocyclic moiety may be unsubstituted or substituted by 1 to 3 methyl groups, one ethyl group or an hydroxyl group;
provided that:
a) when R₆ is hydroxyl, then m is 2; or
b) when R₆ is hydroxyl, then r is 2 to 6; or
c) when R₆ is 2-tetrahydropyranyl, 2-tetrahydrothiopyranyl, 2-tetrahydrofuranyl, or 2-tetrahydrothienyl, then m is 1 or 2; or
d) when R₆ is 2-tetrahydropyranyl, 2-tetrahydrothiopyranyl, 2-tetrahydrofuranyl, or 2-tetrahydrothienyl, then r is 1 to 6;
e) when n is 1 and m is 0, then R₆ is other than H in -(CR₄R₅)ₙO(CR₄R₅)ₘR₆;
X is YR₂, fluorine, NR₄R₅, or formyl amine;
Y is O or S(O)_{m'};
m' is 0, 1, or 2;
X₂ is O or NR₈;
X₃ is hydrogen or X;
R₂ is -CH₃ or -CH₂CH₃ unsubstituted or substituted by 1 or more halogens; s is 0 to 4;
W is alkyl of 2 to 6 carbons, alkenyl of 2 to 6 carbons or alkynyl of 2 to 6 carbons;
R₃ is COOR₁₄, C(O)NR₄R₁₄ or R₁₅;
Z is S(O)ₘ'R₉, OS(O)₂R₉, OR₉, OC(O)NR₇R₇, OC(O)(O)_{q}R₇, O(CR₄R₅)ₙOR₉, or NR₉R₉;
q is 0 or 1;
R₇ is hydrogen or R₉;
R₈ is hydrogen or C₁₋₄ alkyl unsubstituted or substituted by one to three fluorines, or when R₈ and R₁₀ are as -NR₈R₁₀ they may together with the nitrogen form a a 5 to 7 membered ring comprised only of carbon atoms or carbon atoms and at least one heteroatom selected from O/N/or S;
R₉ is C₁₋₁₀ alkyl, C₂₋₁₀ alkenyl, C₃₋₇cycloalkyl, C₄₋₆ cycloalkenyl, aryl, arylalkyl, heteroaryl, heteroarylalkyl, each of which may be unsubstituted or substituted by one or more fluorine atoms, or two R₉ terms appearing as NR₉R₉ may together with the nitrogen form a 5 to 7 membered ring comprised only of carbon atoms or carbon atoms and at least one heteroatom selected from O/N/or S;
R₁₀ is OR₈ or R₈;
R₁₁ is C₁₋₄ alkyl unsubstituted or substituted by one to three fluorines;
R₁₂ is R₁₃, C₃-C₇ cycloalkyl, or an unsubstituted or substituted aryl or heteroaryl group selected from the group consisting of (2-, 3- or 4-pyridyl), pyrimidyl, pyrazolyl, (1- or 2-imidazolyl), pyrrolyl, piperazinyl, piperidinyl, morpholinyl, furanyl, (2- or 3-thienyl), quinolinyl, naphthyl, and phenyl;
R₁₃ is a substituted or unsubstituted heteroaryl group selected from the group consisting of oxazolidinyl, oxazolyl, thiazolyl, pyrazolyl, triazolyl, tetrazolyl, imidazolyl, imidazolidinyl, thiazolidinyl, isoxazolyl, oxadiazolyl, and thiadiazolyl, and where R₁₃ is substituted on R₁₂ or R₁₃ the rings are connected through a carbon atom and each second R₁₃ ring may be unsubstituted or substituted by one or two C₁₋ ₂ alkyl groups unsubstituted or substituted on the methyl with 1 to 3 fluoro atoms;
R₁₄ is hydrogen or R₁₅; or when R₁₀ and R₁₄ are as NR₁₀R₁₄ they may together with the nitrogen form a 5 to 7 membered ring comprised only of carbon atoms or carbon atoms and at least one heteroatom selected from O, N, or S;
R₁₅ is -(CR₄R₅)ₜR₁₂ or C₁₋₆ alkyl wherein the R₁₂ or C₁₋₆ alkyl group is unsubstituted or substituted by one or more times by methyl or ethyl unsubstituted or substituted by one to three fluorines, -F, -Br, -Cl, -NO₂, -Si(R₄)₂, -NR₈R₁₀,-C(O)R₈, -C(O)OR₈, -O(CH₂)_{q}R₈, -CN, -C(O)NR₈R₁₀, -O(CH₂)_{q}C(O)NR₈R₁₀,-O(CH₂)_{q}C(O)R₈, -NR₁₀C(O)NR₈R₁₀, -NR₁₀C(O)R₈, -NR₁₀C(O)OR₉, -NR₁₀C(O)R₁₃, -C(NR₁₀)NR₈R₁₀, -C(NCN)NR₈R₁₀, -C(NCN)SR₁₁, -NR₁₀C(NCN)SR₁₁, -NR₁₀C(NCN)NR₁₀R₈, -NR₁₀S(O)₂R₉, -S(O)ₘ'R₁₁, -NR₁₀C(O)C(O)NR₈R₁₀, -NR₁₀C(O)C(O)R₁₀, or R₁₃;
t is 0, 1, or 2;
provided that:
f) when q is 1 in OC(O)(O)_{q}R₇ , then R₇ is not hydrogen;
g) R₇ is not C₁₋₄ alkyl unsubstituted or substituted by one to three fluorines;
or the pharmaceutically acceptable salts thereof.

2. A compound of Formula (II) 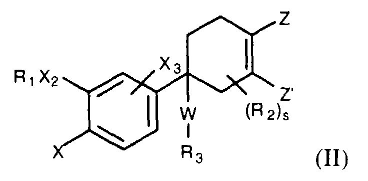 wherein:
R₁ is -(CR₄R₅)ₙC(O)O(CR₄R₅)ₘR₆, -(CR₄R₅)ₙC(O)NR₄(CR₄R₅)ₘR₆,-(CR₄R₅)ₙO(CR₄R₅)ₘR₆, or -(CR₄R₅)ᵣR₆ wherein the alkyl moieties are unsubstituted or substituted with one or more halogens;
m is 0 to 2;
n is 0 to 4;
r is 0 to 6;
R₄ and R₅ are independently hydrogen or a C₁₋₂ alkyl;
R₆ is hydrogen, methyl, hydroxyl, aryl, halo substituted aryl, aryloxyC₁₋₃ alkyl, halo substituted aryloxyC₁₋₃ alkyl, indanyl, indenyl, C₇₋₁₁ polycycloalkyl, tetrahydrofuranyl, furanyl, tetrahydropyranyl, pyranyl, tetrahydrothienyl, thienyl, tetrahydrothiopyranyl, thiopyranyl, C₃₋₆ cycloalkyl, or a C₄₋₆ cycloalkyl containing one or two unsaturated bonds, wherein the cycloalkyl or heterocyclic moiety may be unsubstituted or substituted by 1 to 3 methyl groups, one ethyl group or an hydroxyl group;
provided that:
a) when R₆ is hydroxyl, then m is 2; or
b) when R₆ is hydroxyl, then r is 2 to 6; or
c) when R₆ is 2-tetrahydropyranyl, 2-tetrahydrothiopyranyl, 2-tetrahydrofuranyl, or 2-tetrahydrothienyl, then m is 1 or 2; or
d) when R₆ is 2-tetrahydropyranyl, 2-tetrahydrothiopyranyl, 2-tetrahydrofuranyl, or 2-tetrahydrothienyl, then r is 1 to 6;
e) when n is 1 and m is 0, then R₆ is other than H in -(CR₄R₅)ₙO(CR₄R₅)ₘR₆;
X is YR₂, fluorine, NR₄R₅, or formyl amine;
Y is O or S(O)ₘ';
m' is 0, 1, or 2;
X₂ is O or NR₈;
X₃ is hydrogen or X;
R₂ is -CH₃ or -CH₂CH₃ unsubstituted or substituted by 1 or more halogens;
s is 0 to 4;
W is alkyl of 2 to 6 carbons, alkenyl of 2 to 6 carbons or alkynyl of 2 to 6 carbons;
R₃ is COOR₁₄, C(O)NR₄R₁₄ or R₁₅;
Z is NHR₁₄, S(O)_{m'}R₉, OS(O)₂R₉, OR₉, OC(O)NR₇R₇, OC(O)(O)_{q}R₇, O(CR₄R₅)ₙOR₉, or NR₉R₉;
Z' is C(Y')R₁₄, C(O)OR₁₄, C(Y')NR₁₀R₁₄, C(NR₁₀)NR₁₀R₁₄, CN, C(NOR₈)R₁₄, C(NOR₁₄)R₈, C(NR₈)NR₁₀R₁₄, C(NR₁₄)NR₈R₈ C(NCN)NR₁₀R₁₄, C(NCN)SR₁₁, (2-, 4- or 5-imidazolyl), (3-, 4- or 5-pyrazolyl), (4-or 5-triazolyl[1,2,3]), (3- or 5-triazolyl[1,2,4]), (5-tetrazolyl), (2-, 4- or 5-oxazolyl), (3-, 4- or 5-isoxazolyl), (3- or 5-oxadiazolyl[1,2,4]), (2-oxadiazolyl[1,3,4]), (2-thiadiazolyl[1,3,4]), (2-, 4-, or 5-thiazolyl), (2-, 4-, or 5-oxazolidinyl), (2-, 4-, or 5-thiazolidinyl), or (2-, 4-, or 5-imidazolidinyl); wherein all of the heterocylic ring systems may be optionally substituted one or more times by R₁₄;
Y' is O or S;
q is 0 or 1;
R₇ is hydrogen or R₉;
R₈ is hydrogen or C₁₋₄ alkyl unsubstituted or substituted by one to three fluorines, or when R₈ and R₁₀ are as -NR₈R₁₀ they may together with the nitrogen form a a 5 to 7 membered ring comprised only of carbon atoms or carbon atoms and at least one heteroatom selected from O, N, or S;
R₉ is C₁₋₁₀ alkyl, C₂₋₁₀ alkenyl, C₃₋₇cycloalkyl, C₄₋₆ cycloalkenyl, aryl, arylalkyl, heteroaryl, heteroarylalkyl, each of which may be unsubstituted or substituted by one or more fluorine atoms, or two R₉ terms appearing as NR₉R₉ may together with the nitrogen form a 5 to 7 membered ring comprised only of carbon atoms or carbon atoms and at least one heteroatom selected from O/N/or S;
R₁₀ is OR₈ or R₈;
R₁₁ is C₁₋₄ alkyl unsubstituted or substituted by one to three fluorines;
R₁₂ is R₁₃, C₃-C₇ cycloalkyl, or an unsubstituted or substituted aryl or heteroaryl group selected from the group consisting of (2-, 3- or 4-pyridyl), pyrimidyl, pyrazolyl, (1- or 2-imidazolyl), pyrrolyl, piperazinyl, piperidinyl, morpholinyl, furanyl, (2- or 3-thienyl), quinolinyl, naphthyl, and phenyl;
R₁₃ is a substituted or unsubstituted heteroaryl group selected from the group consisting of oxazolidinyl, oxazolyl, thiazolyl, pyrazolyl, triazolyl, tetrazolyl, imidazolyl, imidazolidinyl, thiazolidinyl, isoxazolyl, oxadiazolyl, and thiadiazolyl, and where R₁₃ is substituted on R₁₂ or R₁₃ the rings are connected through a carbon atom and each second R₁₃ ring may be unsubstituted or substituted by one or two C₁₋ ₂ alkyl groups unsubstituted or substituted on the methyl with 1 to 3 fluoro atoms;
R₁₄ is hydrogen or R₁₅; or when R₁₀ and R₁₄ are as NR₁₀R₁₄ they may together with the nitrogen form a 5 to 7 membered ring comprised only of carbon atoms or carbon atoms and at least one heteroatom selected from O, N, or S;
R₁₅ is -(CR₄R₅)ₜR₁₂ or C₁₋₆ alkyl wherein the R₁₂ or C₁₋₆ alkyl group is unsubstituted or substituted by one or more times by methyl or ethyl unsubstituted or substituted by one to three fluorines, -F, -Br, -Cl, -NO₂, -Si(R₄)₂, -NR₈R₁₀,-C(O)R₈, -C(O)OR₈, -O(CH₂)_{q}R₈, -CN, -C(O)NR₈R₁₀, -O(CH₂)_{q}C(O)NR₈R₁₀,-O(CH₂)_{q}C(O)R₈, -NR₁₀C(O)NR₈R₁₀, -NR₁₀C(O)R₈, -NR₁₀C(O)OR₉, -NR₁₀C(O)R₁₃, -C(NR₁₀)NR₈R₁₀, -C(NCN)NR₈R₁₀, -C(NCN)SR₁₁, -NR₁₀C(NCN)SR₁₁, -NR₁₀C(NCN)NR₁₀R₈, -NR₁₀S(O)₂R₉, -S(O)ₘ'R₁₁, -NR₁₀C(O)C(O)NR₈R₁₀, -NR₁₀C(O)C(O)R₁₀, or R₁₃;
t is 0, 1, or 2;
provided that:
f) when q is 1 in OC(O)(O)_{q}R₇ , then R₇ is not hydrogen;
g) R₇ is not C₁₋₄ alkyl unsubstituted or substituted by one to three fluorines;
or the pharmaceutically acceptable salts thereof.

3. A compound according to claim 1 wherein wherein R₁ is -CH₂-cyclopropyl, -CH₂-C₅₋₆ cycloalkyl, -C₄₋₆ cycloalkyl unsubstituted or substituted by OH, tetrahydrofuran-3-yl, (3- or 4-cyclopentenyl), benzyl or -C₁₋₂ alkyl unsubstituted or substituted by 1 or more fluorines, and -(CH₂)₂₋₄ OH; R₂ is methyl or fluoro-substituted alkyl, W is ethynyl or 1,3-butadiynyl; R₁₅ is an unsubstituted or substituted aryl or heteroaryl ring, X is YR₂, Z is OR₉ or O(S)₂R_{9,} and Z' is COOR₁₄.

4. A compound according to claim 3 wherein R₁ is -CH₂-cyclopropyl, cyclopentyl, 3-hydroxycyclopentyl, methyl or CF₂H; X is YR₂; Y is oxygen; X₂ is oxygen; X₃ is hydrogen; and R₂ is CF₂H or methyl, W is ethynyl or 1,3-butadiynyl, and R₃ is a substituted or unsubstituted pyrimidinyl ring.

5. A compound according to claim 3 which is
4-(3-cyclopentyloxy-4-methoxyphenyl )-4-(4-pyridylethynyl)cyclohex-1-en-1-yl trifluoromethylsulfonate, or
4-(3-cyclopentyloxy-4-methoxyphenyl)-4-(4-pyridylethynyl)-1-methoxycyclohex-1-ene.

6. A pharmaceutical composition comprising a compound of Formula I according to claim 1 and a pharmaceutically acceptable excipient.

## Patentansprüche

1. Verbindung der Formel (I) 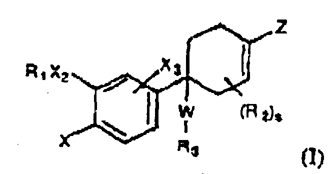
in der R₁ -(CR₄R₅)ₙC(O)O(CR₄R₅)ₘR₆, -(CR₄R₅)ₙC(O)NR₄(CR₄R₅)ₘR₆, -(CR₄R₅)ₙO(CR₄R₅)ₘR₆, oder -(CR₄R₅)ᵣR₆ bedeutet, wobei die Alkylreste unsubstituiert oder mit einem oder mit mehreren Halogenatomen substituiert sind;
m 0 bis 2 ist;
n 0 bis 4 ist;
r 0 bis 6 ist;
R₄ und R₅ unabhängig voneinander ein Wasserstoffatom oder ein C₁₋₂-Alkylrest bedeuten,
R₆ ein Wasserstoffatom, ein Methyl- oder Hydroxyl-, Aryl-, halogensubstituierter Aryl-, AryloxyC₁₋₃-Alkyl-, halogensubstituierter AryloxyC₁₋₃-Alkylrest, ein Indanyl-, Indenyl-, C₇₋₁₁-Polycycloalkyl-, Tetrahydrofuranyl-, Furanyl-, Tetrahydropyranyl-, Pyranyl-, Tetrahydrothienyl-, Thienyl-, Tetrahydrothiopyranyl-, Thiopyranyl-, C₃₋₆-Cycloalkylrest oder ein C₄₋₆-Cycloalkylrest mit einer oder zwei ungesättigten Bindungen bedeutet, wobei der Cycloalkyl- oder heterocyclische Rest unsubstituiert oder mit 1 bis 3 Methylgruppen, einer Ethylgruppe oder einer Hydroxylgruppe substituiert sein kann; mit der Maßgabe, daß:
a) wenn R₆ ein Hydroxylrest bedeutet, m 2 ist; oder
b) wenn R₆ ein Hydroxylrest bedeutet, r 2 bis 6 ist, oder
c) wenn R₆ einen 2-Tetrahydropyranyl-, 2-Tetrahydrothiopyranyl-, 2-Tetrahydrofuranyl- oder 2-Tetrahydrothienylrest bedeutet, m 1 oder 2 ist; oder
d) wenn R₆ einen 2-Tetrahydropyranyl-, 2-Tetrahydrothiopyranyl-, 2-Tetrahydrofuranyl- oder 2-Tetrahydrothienylrest bedeutet, r 1 bis 6 ist;
e) wenn n 1 ist und m 0 ist, dann R₆ in -(CR₄R₅)ₙO(CR₄R₅)ₘR₆ nicht ein Wasserstoffatom bedeutet;
X den Rest YR₂, ein Fluoratom, NR₄R₅ oder ein Formylamin bedeutet;
Y das Atom O oder S(O)_{m'} bedeutet;
m' 0, 1 oder 2 ist;
X₂ O oder NR₈ ist;
X₃ ein Wasserstoffatom oder X bedeutet;
R₂ die Reste -CH₃ oder -CH₂CH₃, unsubstituiert oder mit einem oder mehreren Halogenatomen substituiert, bedeutet;
s 0 bis 4 ist;
W ein Alkylrest mit 2 bis 6 Kohlenstoffatomen, einen Alkenylrest mit 2 bis 6 Kohlenstoffatomen oder einen Alkinylrest mit 2 bis 6 Kohlenstoffatomen bedeutet;
R₃ die Reste COOR₁₄, C(O)NR₄R₁₄ oder R₁₅ bedeutet;
Z die Reste S(O)_{m'}R₉, OS(O)₂R_{9,} OR₉, OC(O)NR₇R₇, OC(O)(O)_{q}R₇, O(CR₄R₅)ₙOR₉ oder NR₉R₉ bedeutet;
q 0 oder 1 ist;
R₇ ein Wasserstoffatom oder R₉ bedeutet;
R₈ ein Wasserstoffatom oder einen unsubstituierten oder mit 1 bis 3 Fluoratomen substituierten C₁₋₄Alkylrest bedeutet, oder wenn R₈ und R₁₀ in der Form -NR₈R₁₀ vorliegen, sie mit dem Stickstoffatom einen fünf- bis siebengliederigen Ring ausbilden können, welcher nur Kohlenstoffatome oder Kohlenstoffatome und mindestens ein Heteroatom, ausgewählt aus O/N/ oder S, umfaßt;
R₉ einen C₁₋₁₀-Alkyl-, C₂₋₁₀-Alkenyl-, C₃₋₇-Cycloalkyl-, C₄₋₆-Cycloalkenyl-, Aryl-, Arylalkyl-, Heteroaryl-, Heteroarylalkylrest bedeutet, wobei jeder davon unsubstituiert oder mit einem oder mehreren Fluoratomen substituiert sein kann, oder wenn zwei Reste R₉ in der Form -NR₉R₉ vorliegen, diese zusammen mit dem Stickstoffatom einen fünf- bis siebengliedrigen Ring ausbilden können, welcher nur Kohlenstoffatome oder Kohlenstoffatome und mindestens ein Heteroatom, ausgewählt aus O/N/ oder S, umfaßt;
R₁₀ die Reste OR₈ oder R₈ bedeutet;
R₁₁ einen unsubstituierten oder mit 1 bis 3 Fluoratomen substituierten C₁₋₄-Alkylrest bedeutet,
R₁₂ den Rest R₁₃, einen C₃-C₇-Cycloalkylrest oder einen substituierten oder unsubstituierten Aryl- oder Heteroarylrest, ausgewählt aus einem (2-, 3- oder 4-Pyridyl)-, Pyrimidyl-, Pyrazolyl-, (1- oder 2-Imidazolyl)-, Pyrrolyl-, Piperazinyl-, Piperidinyl-, Morpholinyl-, Furanyl-, (2- oder 3-Thienyl)-, Chinolinyl-, Naphthyl- und Phenylrest, bedeutet;
R₁₃ einen substituierten oder unsubstituierten Heteroarylrest bedeutet, ausgewählt aus einem Oxazolidinyl-, Oxazolyl-, Thiazolyl-, Pyrazolyl-, Triazolyl-, Tetrazolyl-, Imidazolyl-, Imidazolidinyl-, Thiazolidinyl-, Isoxazolyl-, Oxadiazolyl- und Thiadiazolylrest, und sofern R₁₃ an R₁₂ oder R₁₃ substituiert ist, die Ringe über ein Kohlenstoffatom miteinander verbunden sind und jeder zweite R₁₃-Ring unsubstituiert oder mit 1 oder 2 C₁₋₂-Alkylresten substituiert sein kann, die unsubstituiert oder an der Methylgruppe mit 1 bis 3 Fluoratomen substituiert sein können;
R₁₄ ein Wasserstoffatom oder R₁₅ bedeutet, oder wenn R₁₀ und R₁₄ als NR₁₀R₁₄ vorliegen, diese zusammen mit dem Stickstoff einen fünf- bis siebengliedrigen Ring ausbilden können, der nur aus Kohlenstoffatomen oder aus Kohlenstoffatomen und mindestens einem Heteroatom, ausgewählt aus O,N oder S, besteht;
R₁₅ den Rest -(CR₄R₅)ₜR₁₂ oder einen C₁₋₆-Alkylrest bedeutet, wobei der Rest R₁₂ oder der C₁₋₆-Alkylrest unsubstituiert oder einfach oder mehrfach mit Methyl- oder Ethylresten substituiert ist, welche unsubstituiert oder mit 1 bis 3 Fluoratomen, -F, -Br, -Cl, -NO₂, -Si(R₄)₂, -NR₈R₁₀, -C(O)R₈, -C(O)OR₈, -O(CH₂)_{q}R₈, -CN, -C(O)NR₈R₁₀, -O(CH₂)_{q}C(O)NR₈R₁₀, -O(CH₂)_{q}C(O)R₈,- NR₁₀C(O)NR₈R₁₀, -NR₁₀C(O)R₈, -NR₁₀C(O)OR₉, -NR₁₀C(O)R₁₃, -C(NR₁₀)NR₈ R₁₀, -C(NCN)NR₈R₁₀, -C(NCN)SR₁₁, -NR₁₀C(NCN)SR₁₁, -NR₁₀C(NCN)NR₁₀R₈, -NR₁₀S(O)₂R₉, -S(O)_{m'}R₁₁, -NR₁₀ C(O)C(O)NR₈R₁₀, -NR₁₀C(O)C(O)R₁₀ oder R₁₃ substituiert sind;
t 0, 1 oder 2 ist;
mit der Maßgabe, daß:
f) wenn q in OC(O)(O)_{q}R₇ 1 ist, R₇ kein Wasserstoffatom bedeutet;
g) R₇ keinen unsubstituierten oder mit 1 bis 3 Fluoratomen substituierten C₁₋₄-Alkylrest bedeutet;
oder pharmazeutisch verträgliche Salze davon.

2. Verbindung der Formel (II) 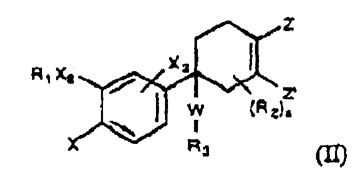
in der R₁ -(CR₄R₅)ₙC(O)O(CR₄R₅)ₘR₆, -(CR₄R₅)ₙC(O)NR₄(CR₄R₅)ₘR₆,
-(CR₄R₅)ₙO(CR₄R₅)ₘR₆, oder -(CR₄R₅)ᵣR₆ bedeutet, wobei die Alkylreste unsubstituiert oder mit einem oder mit mehreren Halogenatomen substituiert sind;
m 0 bis 2 ist;
n 0 bis 4 ist;
r 0 bis 6 ist;
R₄ und R₅ unabhängig voneinander ein Wasserstoffatom oder ein C₁₋₂-Alkylrest bedeuten;
R₆ ein Wasserstoffatom, ein Methyl- oder Hydroxyl-, Aryl-, halogensubstituierter Aryl-, AryloxyC₁₋₃-Alkyl-, halogensubstituierter AryloxyC₁₋₃-Alkylrest, ein Indanyl-, Indenyl-, C₇₋₁₁-Polycycloalkyl-, Tetrahydrofuranyl-, Furanyl-, Tetrahydropyranyl-, Pyranyl-, Tetrahydrothienyl-, Thienyl-, Tetrahydrothiopyranyl-, Thiopyranyl-, C₃₋₆-Cycloalkylrest oder ein C₄₋₆-Cycloalkylrest mit einer oder zwei ungesättigten Bindungen bedeutet, wobei der Cycloalkyl- oder heterocyclische Rest unsubstituiert oder mit 1 bis 3 Methylgruppen, einer Ethylgruppe oder einer Hydroxylgruppe substituiert sein kann; mit der Maßgabe, daß:
a) wenn R₆ ein Hydroxylrest bedeutet, m 2 ist; oder
b) wenn R₆ ein Hydroxylrest bedeutet, r 2 bis 6 ist, oder
c) wenn R₆ einen 2-Tetrahydropyranyl-, 2-Tetrahydrothiopyranyl-, 2-Tetrahydrofuranyl- oder 2-Tetrahydrothienylrest bedeutet, m 1 oder 2 ist; oder
d) wenn R₆ einen 2-Tetrahydropyranyl-, 2-Tetrahydrothiopyranyl-, 2-Tetrahydrofuranyl- oder 2-Tetrahydrothienylrest bedeutet, r 1 bis 6 ist;
e) wenn n 1 ist und m 0 ist, dann R₆ in -(CR₄R₅)ₙO(CR₄R₅)ₘR₆ nicht ein Wasserstoffatom bedeutet;
X den Rest YR₂, ein Fluoratom, NR₄R₅ oder ein Formylamin bedeutet,
Y das Atom O oder S(O)_{m'} bedeutet;
m' 0, 1 oder 2 ist,
X₂ O oder NR₈ ist,
X₃ ein Wasserstoffatom oder X bedeutet;
R₂ die Reste -CH₃ oder -CH₂CH₃, unsubstituiert oder mit einem oder mehreren Halogenatomen substituiert, bedeutet;
s 0 bis 4 ist;
W ein Alkylrest mit 2 bis 6 Kohlenstoffatomen, einen Alkenylrest mit 2 bis 6 Kohlenstoffatomen oder einen Alkinylrest mit 2 bis 6 Kohlenstoffatomen bedeutet;
R₃ die Reste COOR₁₄, C(O)NR₄R₁₄ oder R₁₅ bedeutet;
Z die Reste NHR₁₄, S(O)_{m'}R₉, OS(O)₂R₉, OR₉, OC(O)NR₇R₇, OC(O)(O)_{q}R₇, O(CR₄R₅)ₙOR₉ oder NR₉R₉ bedeutet;
Z' die Reste C(Y')R₁₄, C(O)OR₁₄, C(Y')NR₁₀R₁₄, C(NR₁₀)NR₁₀R₁₄, CN, C(NOR₈)R₁₄, C(NOR₁₄)R₈, C(NR₈)NR₁₀R₁₄, C(NR₁₄)NR₈R₈C(NCN)NR₁₀R₁₄, C(NCN)SR₁₁, (2-, 4- oder 5-Imidazolyl), (3-, 4-, oder 5-Pyrazolyl), (4- oder 5-Triazolyl[1,2,3]), (3- oder 5-Triazolyl[1,2,4]), (5-Tetrazolyl), (2-, 4- oder 5 Oxazolyl), (3-, 4- oder 5-Isoxazolyl), (3- oder 5-Oxadiazolyl[1,2,4]), (2-Oxadiazolyl[1,3,4]), (2-Thiadiazolyl[1,3,4], (2-, 4-, oder 5-Thiazolyl), (2-,4-, oder 5-Oxazolidinyl), (2-,4-, oder 5-Thiazolidinyl), oder (2-,4-, oder 5-Imidazolidinyl) bedeutet; wobei alle heterocyclischen Ringsysteme gegebenenfalls einfach oder mehrfach mit R₁₄ substituiert sein können;
Y' die Atome O oder S bedeutet;
q 0 oder 1 ist;
R₇ ein Wasserstoffatom oder R₉ bedeutet;
R₈ ein Wasserstoffatom oder einen unsubstituierten oder mit 1 bis 3 Fluoratomen substituierten C₁₋₄-Alkylrest bedeutet, oder wenn R₈ und R₁₀ in der Form -NR₈R₁₀ vorliegen, sie mit dem Stickstoffatom einen fünf- bis siebengliederigen Ring ausbilden können, welcher nur Kohlenstoffatome oder Kohlenstoffatome und mindestens ein Heteroatom, ausgewählt aus O/N/ oder S, umfaßt;
R₉ einen C₁₋₁₀-Alkyl-, C₂₋₁₀-Alkenyl-, C₃₋₇-Cycloalkyl-, C₄₋₆-Cycloalkenyl-, Aryl-, Arylalkyl-, Heteroaryl-, Heteroarylalkylrest bedeutet, wobei jeder davon unsubstituiert oder mit einem oder mehreren Fluoratomen substituiert sein kann, oder wenn zwei Reste R₉ in der Form -NR₉R₉ vorliegen, diese zusammen mit dem Stickstoffatom einen fünf- bis siebengliedrigen Ring ausbilden können; welcher nur Kohlenstoffatome oder Kohlenstoffatome und mindestens ein Heteroatom, ausgewählt aus O/N/ oder S, umfaßt;
R₁₀ die Reste OR₈ oder R₈ bedeutet;
R₁₁ einen unsubstituierten oder mit 1 bis 3 Fluoratomen substituierten C₁₋₄-Alkylrest bedeutet,
R₁₂ den Rest R₁₃, einen C₃-C₇-Cycloalkylrest oder einen substituierten oder unsubstituierten Aryl- oder Heteroarylrest, ausgewählt aus einem (2-, 3- oder 4-Pyridyl)-, Pyrimidyl-, Pyrazolyl-, (1- oder 2-Imidazolyl)-, Pyrrolyl-, Piperazinyl-, Piperidinyl-, Morpholinyl-, Furanyl-, (2- oder 3-Thienyl)-, Chinolinyl-, Naphthyl- und Phenylrest, bedeutet;
R₁₃ einen substituierten oder unsubstituierten Heteroarylrest bedeutet, ausgewählt aus einem Oxazolidinyl-, Oxazolyl- Thiazolyl-, Pyrazolyl-, Triazolyl, Tetrazolyl-, Imidazolyl-, Imidazolidinyl-, Thiazolidinyl-, Isoxazolyl-, Oxadiazolyl- und Thiadiazolylrest, und sofern R₁₃ an R₁₂ oder R₁₃ substituiert ist, die Ringe über ein Kohlenstoffatom miteinander verbunden sind und jeder zweite R₁₃-Ring unsubstituiert oder mit 1 oder 2 C₁₋₂-Alkylresten substituiert sein kann, die unsubstituiert oder an der Methylgruppe mit 1 bis 3 Fluoratomen substituiert sein können;
R₁₄ ein Wasserstoffatom oder R₁₅ bedeutet, oder wenn R₁₀ und R₁₄ als NR₁₀R₁₄ vorliegen, diese zusammen mit dem Stickstoff einen fünf- bis siebengliedrigen Ring ausbilden können, der nur aus Kohlenstoffatomen oder aus Kohlenstoffatomen und mindestens einem Heteroatom, ausgewählt aus O,N oder S, besteht;
R₁₅ den Rest -(CR₄R₅)ₜR₁₂ oder einen C₁₋₆-Alkylrest bedeutet wobei der Rest R₁₂ oder der C₁₋₆-Alkylrest unsubstituiert oder einfach oder mehrfach mit Methyl- oder Ethylresten substituiert ist, welche unsubstituiert oder mit 1 bis 3 Fluoratomen, -F, -Br, -Cl, -NO₂, -Si(R₄)₂, -NR₈R₁₀, -C(O)R₈, -C(O)OR₈, -O(CH₂)_{q}R₈, -CN, -C(O)NR₈R₁₀, -O(CH₂)_{q}C(O)NR₈R₁₀, -O(CH₂)_{q}C(O)R₈,- NR₁₀C(O)NR₈R₁₀, -NR₁₀C(O)R₈, -NR₁₀C(O)OR₉, -NR₁₀C(O)R₁₃, -C(NR₁₀)NR₈ R₁₀, -C(NCN)NR₈R₁₀, -C(NCN)SR₁₁, -NR₁₀C(NCN)SR₁₁, -NR₁₀C(NCN)NR₁₀R₈, -NR₁₀S(O)₂R₉, -S(O)_{m'}R₁₁, -NR₁₀ C(O)C(O)NR₈R₁₀, -NR₁₀C(O)C(O)R₁₀ oder R₁₃ substituiert sind;
t 0, 1 oder 2 ist;
mit der Maßgabe, daß:
f) wenn q in OC(O)(O)_{q}R₇ 1 ist, R₇ kein Wasserstoffatom bedeutet;
g) R₇ keinen unsubstituierten oder mit 1 bis 3 Fluoratomen substituierten C₁₋₄-Alkylrest bedeutet;
oder pharmazeutisch verträgliche Salze davon.

3. Verbindung nach Anspruch 1, in der R₁ die Reste -CH₂-Cyclopropyl, -CH₂-C₅₋₆-Cycloalkyl, -C₄₋₆Cycloalkyl, unsubstituiert oder substituiert mit einer OH-Gruppe, Tetrahydrofuran-3-yl, (3- oder 4-Cyclopentenyl) oder Benzyl bedeutet, oder einen unsubstituierten oder mit einem oder mehreren Fluoratomen substituierten C₁₋₂-Alkylrest und den Rest -(CH₂)₂₋₄OH bedeutet;
R₂ einen methyl- oder fluorsubstituierten Alkylrest bedeutet;
W einen Ethinyl- oder 1,3-, Butadiynylrest bedeutet;
R₁₅ einen unsubstituierten oder substituierten Aryl- oder Heteroarylring bedeutet;
X den Rest YR₂, Z die Reste OR₉ oder O(S)₂R₉ und Z' den Rest COOR₁₄ bedeutet.

4. Verbindung nach Anspruch 3, in der R₁ die Reste -CH₂-Cyclopropyl-, Cyclopentyl, 3-Hydoxycyclopentyl, Methyl oder -CF₂H bedeutet;
X den Rest YR₂ bedeutet;
Y Sauerstoff bedeutet;
X₂ Sauerstoff bedeutet;
X₃ ein Wasserstoffatom bedeutet und
R₂ die Reste CF₂H oder Methyl bedeutet;
W einen Ethinyl- oder 1,3-Butadiynylrest bedeutet und
R₃ einen substituierten oder unsubstituierten Pyrimidinylring bedeutet.

5. Verbindung nach Anspruch 3, die
4-(3-Cyclopentyloxy-4-methoxyphenyl)-4-(4-pyridylethinyl)cyclohex-1-en-1-yl-trifluormethylsulfonat oder
4-(3-Cyclopentyloxy-4-methoxyphenyl)-4-(4-pyridylethinyl)-1-methoxycyclohex-1-en ist.

6. Arzneimittel umfassend eine Verbindung der Formel (I) nach Anspruch 1 und einen pharmazeutisch verträglichen Exzipienten.

## Revendications

1. Composé de formule (I) 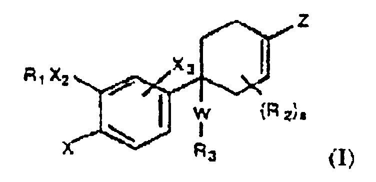 dans laquelle
R₁ est un groupe -(CR₄R₅)ₙC(O)O(CR₄R₅)ₘR₆, -(CR₄R₅)ₙC(O)NR₄(CR₄R₅)ₘR₆, -(CR₄R₅)ₙO(CR₄R₅)ₘR₆ ou -(CR₄R₅)ᵣR₆, où les fragments alkyle sont non substitués ou substitués avec un ou plusieurs atomes d'halogènes ;
m a une valeur de 0 à 2 ;
n a une valeur de 0 à 4 ;
r a une valeur de 0 à 6 ;
R₄ et R₅ représentent, indépendamment, l'hydrogène ou un groupe alkyle en C₁ ou C₂ ;
R₆ est l'hydrogène, un groupe méthyle, hydroxyle, aryle, aryle à substituant halogéno, aryloxy-(alkyle en C₁ à C₃), aryloxy-(aryle en C₁ à C₃) à substituant halogéno, indanyle, indényle, polycycloalkyle en C₇ à C₁₁, tétratydrofurannyle, furannyle, tétrahydropyrannyle, pyrannyle, tétrahydrothiényle, thiényle, tétrahydrothiopyrannyle, thiopyrannyle, cycloalkyle en C₃ à C₆ ou un groupe cycloalkyle en C₄ à C₆ contenant une ou deux liaisons de non-saturation, le fragment cycloalkyle ou le fragment hétérocyclique pouvant être non substitué ou substitué par 1 à 3 groupes méthyle, un groupe éthyle ou un groupe hydroxyle ;
sous réserve que :
a) lorsque R₆ est un groupe hydroxyle, m soit égal à 2 ; ou bien
b) lorsque R₆ est un groupe hydroxyle, r ait une valeur de 2 à 6 ; ou
c) lorsque R₆ est un groupe 2-tétrahydropyrannyle, 2-tétrahydrothiopyrannyle, 2-tétrahydrofurannyle ou 2-tétrahydrothiényie, m ait la valeur 1 ou 2 ; ou
d) lorsque R₆ est un groupe 2-tétrahydropyrannyle, 2-tétrahydrothiopyrannyle, 2-tétrahydrofurannyle ou 2-tétrahydrothiényle, r ait une valeur de 1 à 6 ;
e) lorsque n est égal à 1 et m est égal à 0, R₆ représente autre chose que H dans -(CR₄R₅)ₙO(CR₄R₅)ₘR₆ ;
X est un groupe YR₂, du fluor, un groupe NR₄R₅ ou formylamine ;
Y représente O ou S(O)_{m'} ;
m' a la valeur 0, 1 ou 2 ;
X₂ représente O ou NR₈ ;
X₃ représente l'hydrogène ou X ;
R₂ est un groupe -CH₃ ou -CH₂CH₃ non substitué ou substitué par un ou plusieurs halogènes ;
s a une valeur de 0 à 4 ;
W est un groupe alkyle de 2 à 6 atomes de carbone, alcényle de 2 à 6 atomes de carbone ou alcynyle de 2 à 6 atomes de carbone ;
R₃ est un groupe COOR₁₄, C(O)NR₄R₁₄ ou R₁₅ ;
Z est un groupe S(O)_{m'}R₉, OS(O)₂R₉, OR₉, OC(O)NR₇R₇, OC(O)(O)_{q}R₇, O(CR₄R₅)ₙOR₉ ou NR₉R₉ ;
q a la valeur 0 ou 1 ;
R₇ est l'hydrogène ou R₉ ;
R₈ est l'hydrogène ou un groupe alkyle en C₁ à C₄ non substitué ou substitué par un ou à trois atomes de fluor, ou bien lorsque R₈ et R₁₀ sont inclus dans un groupe -NR₈R₁₀, ils peuvent former conjointement avec l'azote un noyau pentagonal à heptagonal constitué uniquement d'atomes de carbone ou d'atomes de carbone et d'au moins un hétéroatome choisi entre O/N/ ou S ;
R₉ est un groupe alkyle en C₁ à C₁₀, alcényle en C₂ à C₁₀, cycloalkyle en C₃ à C₇, cycloalcényle en C₄ à C₆, aryle, arylalkyle, hétéroaryle, hétéroarylalkyle dont chacun peut être non substitué ou substitué par un ou plusieurs atomes de fluor, ou bien deux termes R₉ apparaissant dans un groupe NR₉R₉ peuvent former conjointement avec l'azote un noyau pentagonal à heptagonal constitué uniquement d'atomes de carbone ou d'atomes de carbone et d'au moins un hétéroatome choisi entre O/N/ ou S ;
R₁₀ représente OR₈ ou R₈ ;
R₁₁ représente un groupe alkyle en C₁ à C₄ non substitué ou substitué par un ou deux atomes de fluor ;
R₁₂ représente R₁₃, un groupe cycloalkyle en C₃ à C₇ ou un groupe aryle ou hétéroaryle non substitué ou substitué choisi entre (2-, 3- ou 4-pyridyle), pyrimidyle, pyrazolyle, (1- ou 2-imidazolyle), pyrrolyle, pipérazinyle, pipéridinyle, morpholinyle, furannyle, (2- ou 3-thiényle), quinolinyle, naphtyle et phényle ;
R₁₃ est un groupe hétéroaryle substitué ou non substitué choisi dans le groupe consistant en oxazolidinyle, oxazolyle, thiazolyle, pyrazolyle, triazolyle, tétrazolyle, imidazolyle, imidazolidinyle, thiazolidinyle, isoxazolyle, oxadiazolyle et thiadiazolyle, et lorsque R₁₃ est substitué sur R₁₂ ou R₁₃, les normaux sont liés par un atome de carbone et un noyau R₁₃ sur deux peut être non substitué ou substitué par un ou deux groupes alkyle en C₁ ou C₂ non substitués ou substitués sur le radical méthyle avec 1 à 3 atomes de fluor ;
R₁₄ est l'hydrogène ou R₁₅ ; ou bien lorsque R₁₀ et R₁₄ apparaissent comme groupe NR₁₀R₁₄, ils peuvent former conjointement avec l'azote un noyau pentagonal à heptagonal constitué uniquement d'atomes de carbone ou d'atomes de carbone et d'au moins un hétéroatome choisi entre O, N ou S ;
R₁₅ est un groupe -(CR₄R₅)ₜR₁₂ ou alkyle en C₁ à C₆, R₁₂ ou le groupe alkyle en C₁ à C₆ étant non substitué ou substitué une ou plusieurs fois par un radical méthyle ou éthyle substitué ou non substitué par un ou trois atomes de fluor, -F, -Br, -Cl, -NO₂, -Si(R₄)₂, -NR₈R₁₀, -C(O)R₈, -C(O)OR₈, -O(CH₂)_{q}R₈, -CN, -C(O)NR₈R₁₀, O(CH₂)_{q}C(O)NR₈R₁₀, -O(CH₂)_{q}C(O)R₈, -NR₁₀C(O)NR₈R₁₀, -NR₁₀C(O)R₈, -NR₁₀C(O)OR₉, -NR₁₀C(O)R₁₃, -C(NR₁₀)NR₈R₁₀, -C(NCN)NR₈R₁₀, -C(NCN)SR₁₁, -NR₁₀C(NCN)SR₁₁, -NR₁₀C(NCN)NR₁₀R₈, -R₁₀S(C)₂R₉, -S(O)ₘR₁₁, -NR₁₀C(O)C(O)NR₈R₁₀, -NR₁₀C(O)C(O)R₁₀ ou R₁₃ ;
t a la valeur 0, 1 ou 2
sous réserve que :
f) lorsque q est égal à 1 dans OC(O) (O)_{q}R₇, R₇ ne soit pas de l'hydrogène ;
g) R₇ ne soit pas un groupe alkyle en C₁ à C₄ non substitué ou substitué par un à trois atomes de fluor ;
ou les sels acceptables du point de vue pharmaceutique de ce composé.

2. Composé de formule (II) 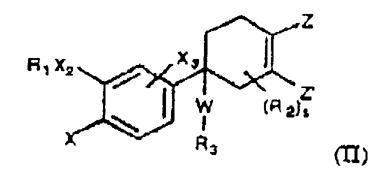 dans laquelle
R₁ est un groupe (CR₄R₅)ₙC(O)O(CR₄R₅)ₘR₆, -(CR₄R₅)ₙC(O)NR₄(CR₄R₅)ₘR₆, -(CR₄R₅)ₙO(CR₄R₅)ₘR₆ ou -(CR₄R₅)ᵣR₆, dans lequel les fragments alkyle sont non substitués ou substitués avec un ou plusieurs atomes halogènes ;
m a une valeur de 0 à 2
n a une valeur de 0 à 4 ;
r a une valeur de 0 à 6 ;
R₄ et R₅ représentent, indépendamment, l'hydrogène ou un groupe alkyle en C₁ ou C₂ ;
R₆ est l'hydrogène, un groupe méthyle, hydroxyle, aryle, aryle à substituant halogéno, aryloxy-(alkyle en C₁ à C₃), aryloxy-(alkyle en C₁ à C₃) à substituant halogéno, indanyle, indényle, polycycloalkyle en C₇ à C₁₁, tétrahydrofurannyle, furannyle, tétrahydropyrannyle, pyrannyle, tétrahydrothiényle, thiényle, tétrahydrothiopyrannyle, thiopyrannyle, cycloalkyle en C₃ à C₆ ou un groupe cycloalkyle en C₄ à C₆ contenant une ou deux liaisons de non-saturation, le fragment cycloalkyle ou hétérocyclique pouvant être non substitué ou substitué par 1 à 3 groupes méthyle, un groupe éthyle ou un groupe hydroxyle ;
sous réserve que :
a) lorsque R₆ est un groupe hydroxyle, m soit égal à 2 ; ou bien
b) lorsque R₆ est un groupe hydroxyle, r ait une valeur de 2 à 6 ; ou bien
c) lorsque R₆ est un groupe 2-tétrahydropyrannyle, 2-tétrahydrothiopyrannyle, 2-tétrahydrofurannyle ou 2-tétrahydrothiényle, m ait la valeur 1 ou 2 ; ou bien
d) lorsque R₆ est un groupe 2-tétrahydropyrannyle, 2-tétrahydrothiopyrannyle, 2-tétrahydrofurannyle ou 2-tétrahydrothiényle, r ait une valeur de 1 à 6 ;
e) lorsque n est égal à 1 et m est égal à 0, R₆ représente autre chose que H dans -(CR₄R₅)ₙO(CR₄R₅)ₘR₆ ;
X représente un groupe YR₂, le fluor, un groupe NR₄R₅ ou formylamine ;
Y représente O ou S(O)_{m'} ;
m' a la valeur 0, 1 ou 2 ;
X₂ représente O ou un groupe NR₈ ;
X₃ représente l'hydrogène ou X ;
R₂ représente un groupe -CH₃ ou -CH₂CH₃ non substitué ou substitué par un ou plusieurs halogènes ;
s a une valeur de 0 à 4 ;
W est un groupe alkyle de 2 à 6 atomes de carbone, alcényle de 2 à 6 atomes de carbone ou alcynyle de 2 à 6 atomes de carbone ;
R₃ est un groupe COOR₁₄, C(O)NR₄R₁₄ ou R₁₅ ;
Z est un groupe NHR₁₄, S(O)_{m'}R₉, OS(O)₂R₉, OR₉, OC(O)NR₇R₇, OC(O) (O)_{q}R₇, O(CR₄R₅)ₙOR₉ ou NR₉R₉ ;
Z' est un groupe C(Y')R₁₄, C(O)OR₁₄, C(Y')NR₁₀R₁₄, C(NR₁₀)NR₁₀R₁₄, CN, C(NOR₈)R₁₄, C(NOR₁₄)R₈, C(NR₈)NR₁₀R₁₄, C(NR₁₄)NR₈R₈, C(NCN)NR₁₀R₁₄, C(NCN)SR₁₁, (2-, 4- ou 5-imidazolyle), (3-, 4- ou 5-pyrazolyle), (4- ou 5-triazolyle[1.2.3]), (3- ou 5-triazolyle[1.2.4]), (5-tétrazole), (2-, 4- ou 5-oxazolyle), (3-, 4- ou 5-isoxazolyle), (3- ou 5-oxadiazolyle[1.2.4]), (2-oxadiazolyle[1.3.4]), (2-thiadiazolyle[1.3.4]), (2-, 4- ou 5-thiazolyle), (2-, 4- ou 5-oxazolidinyle), (2-, 4- ou 5-thiazolidinyle) ou (2-, 4- ou 5-imidazolidinyle) ; où tous les systèmes de noyaux hétérocycliques peuvent facultativement être subsitutés une ou plusieurs fois par R₁₄ ;
Y' représente O ou S ;
q a la valeur 0 ou 1 ;
R₇ est l'hydrogène ou R₉ ;
R₈ est l'hydrogène ou un groupe alkyle en C₁ à C₄ non substitué ou substitué par un à trois atomes de fluor, ou bien lorsque R₈ et R₁₀ apparaissent dans un groupe -NR₈R₁₀, ils peuvent former conjointement avec l'atome d'azote un noyau pentagonal à heptagonal constitué uniquement d'atomes de carbone ou d'atomes de carbone et d'au moins un hétéroatome choisi entre O, N ou S ;
R₉ est un groupe alkyle en C₁ à C₁₀, alcényle en C₂ à C₁₀, cycloalkyle en C₃ à C₇, cycloalcényle en C₄ à C₆, aryle, arylalkyle, hétéroaryle, hétéroarylalkyle dont chacun peut être non substitué ou substitué par un ou plusieurs atomes de fluor, ou bien deux termes R₉ apparaissant dans un groupe NR₉R₉ peuvent former conjointement avec l'azote un noyau pentagonal à heptagonal constitué uniquement d'atomes de carbone ou d'atomes de carbone et d'au moins un hétéroatome choisi entre C/N/ ou S ;
R₁₀ représente OR₈ ou R₈ ;
R₁₁ est un groupe alkyle en C₁ à C₄ non substitué ou substitué par un à trois atomes de fluor ;
R₁₂ est un groupe R₁₃, cycloalkyle en C₃ à C₇ ou un groupe aryle ou hétéroaryle non substitué ou substitué choisi dans le groupe consistant en (2-, 3- ou 4-pyridyle), pyrimidyle, pyrazolyle, (1- ou 2-imidazolyle), pyrrolyle, pipérazinyle, pipéridinyle, morpholinyle, furannyle, (2- ou 3-thiényle), quinolinyle, naphtyle et phényle ;
R₁₃ est un groupe hétéroaryle substitué ou non substitué choisi dans le groupe consistant en oxazolidinyle, oxazolyle, thiazolyle, pyrazolyle, triazolyle, tétrazolyle, imidazolyle, imidazolidinyle, thiazolidinyle, isoxazolyle, oxadiazolyle et thiadiazolyle, et lorsque R₁₃ est substitué sur R₁₂ ou R₁₃, les noyaux sont liés par un atome de carbone et un noyau R₁₃ sur deux peut être non substitué ou substitué par un ou deux groupes alkyle en C₁ ou C₂ non substitués ou substitués sur le radical méthyle avec 1 à 3 atomes de fluor ;
R₁₄ est l'hydrogène ou un groupe R₁₅ ; ou bien lorsque R₁₀ et R₁₄ apparaissent sous la forme NR₁₀R₁₄, ils peuvent former conjointement avec l'azote un noyau pentagonal à heptagonal constitué uniquement d'atomes de carbone ou d'atomes de carbone et d'au moins un hétéroatome choisi entre O, N ou S ;
R₁₅ est un groupe (CR₄R₅)ₜR₁₂ ou alkyle en C₁ à C₆, où R₁₂ ou le groupe alkyle en C₁ à C₆ est non substitué ou substitué une ou plusieurs fois par un radical méthyle ou éthyle non substitué ou substitué par un à trois atomes de fluor, -F, -Br, -Cl, -NO₂, -Si(R₄)₂, -NR₈R₁₀, -C(O)R₈, -C(O)OR₈, -O(CH₂)_{q}R₈, -CN, -C(O)NR₈R₁₀, -O(CH₂)_{q}C(O)NR₈R₁₀, -O(CH₂)_{q}C(O)R₈, -NR₁₀C(O)NR₈R₁₀, -NR₁₀C(O)R₈, -NR₁₀C(O)OR₉, -NR₁₀C(O)R₁₃, -C(NR₁₀)NR₈R₁₀, -C(NCN)NR₈R₁₀, -C(NCN)SR₁₁, -NR₁₀C(NCN)SR₁₁, -NR₁₀C(NCN)NR₁₀R₈, -NR₁₀S(O)₂R₉, -S(O)ₘR₁₁, -NR₁₀C(O)C(O)NR₈R₁₀, -NR₁₀C(O)C(O)R₁₀ ou R₁₃ ;
t a la valeur 0, 1 ou 2 ;
sous réserve que :
f) lorsque q est égal à 1 dans OC(O)(O)_{q}R₇, R₇ ne soit pas de l'hydrogène ;
g) R₇ ne soit pas un groupe alkyle en C₁ à C₄ non substitué ou substitué par un à crois atomes de fluor ;
ou les sels pharmaceutiquement acceptables de ce composé.

3. Composé suivant la revendication 1, dans lequel R₁ est un groupe -CH₂-, cyclopropyle, -CH₂-(cycloalkyle en C₅ ou C₆), -cycloalkyle en C₄ à C₆ non substitué ou substitué par un radical OH, tétrahydrofuranne-3-yle, (3- ou 4-cyclopentényle), benzyle ou -alkyle en C₁ ou C₂ non substitué ou substitué par un ou plusieurs atomes de fluor, et -(CH₂)₂₋₄OH ; R₂ est un groupe méthyle ou alkyle à substituant fluoro, W est un groupe éthynyle ou 1,3-butadiynyle ; R₁₅ est un noyau aryle ou hétéroaryle non substitué ou substitué, X représente YR₂, Z représente OR₉ ou O(S)₂R₉ et Z' représente COOR₁₄.

4. Composé suivant la revendication 3, dans lequel R₁ est un groupe -CH₂-cyclopropyle, cyclopentyle, 3-hydroxycyclopentyle, méthyle ou CF₂H ; X représente YR₂ ; Y représente l'oxygène ; X₂ représente l'oxygène ; X₃ représente l'hydrogène ; et R₂ est un groupe CF₂H ou méthyle, W est un groupe éthynyle ou 1,3-butadiynyle, et R₃ est un noyau pyrimidinyle substitué ou non substitué.

5. Composé suivant la revendication 3, qui est
le trifluorométhylsulfonate de 4-(3-cyclopentyloxy-4-méthoxyphényl)-4-(4-pyridyléthynyl)cyclohex-1-ène-1-yle, ou
le 4-(3-cyclopentyloxy-4-méthoxyphényl)-4-(4-pyridyléthynyl)-1-méthoxycyclohex-1-ène.

6. Composition pharmaceutique comprenant un composé de formule I suivant la revendication 1 et un excipient acceptable du point de vue pharmaceutique.
